(19) Europäisches Patentamt
European Patent Office
Office européen des brevets

(11) EP 1 654 013 B1

(12) **EUROPÄISCHE PATENTSCHRIFT**

(45) Veröffentlichungstag und Bekanntmachung des Hinweises auf die Patenterteilung:
**11.11.2009 Patentblatt 2009/46**

(21) Anmeldenummer: **04726466.8**

(22) Anmeldetag: **08.04.2004**

(51) Int Cl.:
***A61L 15/44*** *(2006.01)* ***A61L 15/40*** *(2006.01)*

(86) Internationale Anmeldenummer:
**PCT/EP2004/003772**

(87) Internationale Veröffentlichungsnummer:
**WO 2004/089431 (21.10.2004 Gazette 2004/43)**

(54) **ANTIMIKROBIELL AUSGERÜSTETE MATERIALIEN**

ANTIMICROBIAL MATERIALS

MATIERES A TRAITEMENT ANTIMICROBIEN

(84) Benannte Vertragsstaaten:
**AT BE BG CH CY CZ DE DK EE ES FI FR GB GR HU IE IT LI LU MC NL PL PT RO SE SI SK TR**

(30) Priorität: **09.04.2003 DE 10316156**
**09.10.2003 US 681204**
**09.10.2003 US 681236**

(43) Veröffentlichungstag der Anmeldung:
**10.05.2006 Patentblatt 2006/19**

(73) Patentinhaber: **Beiersdorf AG**
**20253 Hamburg (DE)**

(72) Erfinder:
- **BERG, Thorsten**
  **20149 Hamburg (DE)**
- **JÄNICHEN, Dr. Jan**
  **22143 Hamburg (DE)**
- **SCHINK, Michael**
  **22609 Hamburg (DE)**
- **BOGDAHN, Michael**
  **21614 Buxtehude-Neukloster (DE)**
- **GÄDE, Christian**
  **21629 Neu Wulmstorf (DE)**
- **QUANDT, Jürgen, Christian**
  **25336 Klein Nordende (DE)**
- **KARTHEUS, Holger**
  **20255 Hamburg (DE)**
- **HARTKOPF, Carsten**
  **22337 Hamburg (DE)**
- **WÖLLER, Karl-Heinz**
  **20257 Hamburg (DE)**
- **MEYER-INGOLD, Wolfgang**
  **22457 Hamburg (DE)**

(56) Entgegenhaltungen:
**EP-A- 1 116 698** **EP-A- 1 116 700**
**WO-A2-96/24364** **DE-A- 10 213 632**
**US-A- 5 470 585**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 654 013 B1

## Beschreibung

Die Erfindung betrifft Materialien, insbesondere selbstklebend ausgerüstete und antimikrobiell wirksame Wundauflagen, Kosmetika, insbesondere entzündungslindernde Emulsionen, desinfizierende Reinigungszubereitungen sowie antimikrobiell wirksame Hautauflagen, Pads oder Tücher.

Die Kombination von Materialien, wie beispielsweise Polyurethan als Wundauflage, mit den bestimmten silberhaltigen Gläsern generiert eine desinfizierende, hautberuhigende oder antimikrobielle Wirkung und gewährleistet gleichzeitig eine hohe Produktstabilität und eine hohe Verfärbungsstabilität der Materialien gegenüber äußeren Einflüssen.

Die Behandlung und Heilung von bakteriell kontaminierten beziehungsweise von infizierter Haut oder Wunden stellt eine große Herausforderung für die Medizin und Naturwissenschaften dar. Vor allem bei schwer heilenden und chronischen Wunden tritt oftmals eine Besiedelung durch verschiedenste Mikroorganismen auf, die den Verlauf der Heilung stark verzögern oder manchmal auch völlig unterbinden. Aber auch bei akuten Wunden, die durch Traumata, chirurgische Eingriffe oder auch nur einfache Verletzungen entstanden sind, ist ein Eindringen von pathogenen Keimen nicht in jedem Fall auszuschließen.

Durch das Eindringen der Keime findet eine Kolonisation der Wunde mit Mikroorganismen statt. Bei einer Besiedlung der Wunde mit mehr als $10^5$ KBE/g spricht man von einer infizierten Wunde (M.C.Robson "Clinical Research can improve the outcome of treatment of problem wounds : Infection as a paradigm", 8th Annual Meeting of the ETRS, Copenhagen, DK, August 27-30, 1998). Durch die massive Besiedlung des Wundmilieus mit Mikroorganismen kann es zu einer massiven Störung des Heilungsverlaufes kommen, der in letzter Konsequenz zur Lethalität führen kann. Häufige Auslöser von bakteriellen Wundinfektionen gehören zu den Gattungen Pseudomonas, Staphylococcus, Clostridium und bei den Hefen und Schimmelpilzen zu den Gattungen Candida und Aspergillus. Eine Eingrenzung auf wenige Arten ist nicht möglich, da viele der Mikroorganismen als opportunistische Krankheitserreger anzusehen sind.

Es sind verschiedene Möglichkeiten beschrieben, Mikroorganismen aus dem kontaminierten oder infizierten Gewebe einer Wunde zu entfernen, beziehungsweise darin abzutöten. Außer durch die orale Gabe von Antibiotika kann das Entfernen von pathogenen Mikroorganismen aus einer Wunde nach dem Stand der Technik durch die topische Anwendung eines Desinfektionsmittels oder eines Antibiotikums erreicht werden. Ferner sind Antiseptika und Antibiotika zytotoxisch, und darüber hinaus haben viele pathogene Stämme Resistenzen gegen Antibiotika entwickelt. Dass sogar auch Resistenz-Entwicklung gegenüber einem Antiseptikum möglich ist, wurde am Beispiel Triclosan resistenter E. coli Bakterien berichtet (McMurry LM et al (1998) FEMS Microbiol Lett 166(2): 305-9, Cookson BD et al (1991) Lancet 337 (8756): 1548-9; Uhl S (1993) Lancet 342(8865): 248). Maßgeblich dabei war vor allem der weitverbreitete und prophylaktische Einsatz von Triclosan (Irgasan®) in Seifen, Deos, Textilien und Kunststoffen.

Hinlänglich bekannt und beispielhaft zur antimikrobiellen und/oder vorbeugenden Therapie von kontaminierten beziehungsweise infizierten Wunden ist die Verwendung von Oxidantien (zum Beispiel Jodtinktur) oder Antiseptika (zum Beispiel Salben mit Silbersulfadiazin). Auch in Form von entsprechend antimikrobiell ausgerüsteten oder imprägnierten Wundauflagen und Wundversorgungsmaterialien kommen derartige Agentien zum Einsatz. Bekannt ist dabei auch der Einsatz von silberhaltigen Zeolithen.

Eine Übersicht der aus dem Stand der Technik bekannten antimikrobiell wirksamen silberhaltigen Wundversorgungsmaterialien liefert DE-A1-19958458. DE-A1-19958458 offenbart Wundauflagen, die aus einem synthetischen Polymermaterial bestehen, das metallionenhaltige Zeolithe enthält.

Bekannt sind darüber hinaus aus der EP-A1-1116698 und EP-A1-1116700 silberhaltige Gläser, die eine antimikrobielle Wirksamkeit aufweisen. Diese werden jedoch nur in thermoplastischen Polymeren eingebettet, die im Haushalts- und Sanitärbereich in verschiedensten Formen wie Wandtapeten, Schneidbretter o. ä. eingesetzt werden.

Neben der antimikrobiellen Wirksamkeit steht auch die Schaffung eines feuchten Wundmilieus, das im Gegensatz zur traditionellen trockenen Wundbehandlung, wie zum Beispiel mittels Mullkompressen, den natürlichen Abläufen der Wundheilung physiologische und damit bessere Konditionen bietet, im Zentrum der Entwicklung der antimikrobiellen Wundheilung.

EP-A1-1159972 offenbart ein Verbandmaterial bestehend aus einer selbstklebenden Hydrocolloid-Zusammensetzung, das ein feuchtes Wundmilieu ermöglicht und ein antimikrobielles Agenz, enthaltend Silber, Kupfer und Zink, enthält.

Auf dem Markt ist unter dem Namen Contreet-H® der Firma Coloplast ein antibakterieller Hydrokolloidverband erhältlich, der eine feuchte Wundbehandlung und eine antibakterielle Wirkung durch Imprägnierung mit ionischem Silber ermöglicht.

Entsprechende Verbände sind in WO 00/09173 und US 5681575 sowie in WO 02/062403 und WO 02/078755 beschrieben.

WO 02/062403 beschreibt eine antimikrobielle Wundauflage, die in der Klebmatrix einen silberhaltigen Komplex, umfassend mindestens ein Element der Gruppe IV des PSE enthält. Als bevorzugte Elemente werden Titan, Hafnium oder Zirkonium genannt, wobei der Komplex bevorzugt ein Phosphatkomplex darstellt.

Die Silberionen, in einem Anteil von 0,01 bis 30 mg/cm$^2$ Wundauflage, werden nur bei Kontakt mit ionischer Lösung

freigesetzt. Dabei kommen als Klebmassen nur solche in Frage, die die Silberfreisetzung und antimikrobielle Aktivität des Silbers nicht herabsetzen. Als Beispiel wird ein Polyurethanschaum als Matrix beschreiben, wobei die Schaumcharakteristik im Hinblick auf die Freisetzungsrate zwingend ist.

WO 02/078755 beschreibt eine antimikrobielle Wundauflage mit einer Silberfreisetzung von 50 bis 10.000 $\mu g/cm^2$ Wundauflage sowie einer Aufnahmekapazität an Wundexudat von mehr als 0,09 $g/cm^2$.
Die Silberverbindungen liegen dabei, wie in WO 02/062403, in komplexer Form mit Elementen der 4. Gruppe des PSE vor. Bevorzugt ist auch hier ein Zirkoniumphosphatkomplex.

Die in WO 02/078755 angegebene sehr weit gefasste Freisetzungsrate liegt dabei in Bereichen, die ebenfalls mit den bekannten silberhaltigen antimikrobiellen Wundauflagen des Standes der Technik erreicht werden.

US 6143318 beschreibt ein Verfahren zur Bekämpfung der Wundinfektionen umfassend ein Wundauflage enthaltend ein wasserlösliches Glas, welches Silber und Kupfer, Magnesium oder Zink freisetzen kann.
GB 2178422 beschreibt Prothesenmaterial, das Glas enthält, umfassend CaO, ZnO, MgO, $P_2O_5$, $Na_2O$ und $K_2O$ in einer speziellen Zusammensetzung, wobei nicht mehr als 5 mol% des $P_2O_5$ durch $Ag_2O$, FeO, CuO, $TiO_2$ oder $ZrO_2$ ersetzt werden kann. DE 10213632 beschreibt ein antimikrobielles, entzündungshemmendes, wundheilendes Glas einer spezifischen Zusammensetzung, das zusätzlich auch Ag, Cu und/oder Zn enthalten kann.

US-A-5470585 und WO 96/24364 A offenbaren jeweils eine Glaszusammensetzung, die weniger als 5 mol% $SiO_2$, kein $Al_2O_3$, $Na_2O$ immer über 10 mol% und Silber als Orthophosphat umfasst. Darüber hinaus sind die Materialien, die das Silberorthophosphat enthalten, zumeist keine Materialien, die auf die Haut aufzutragen oder zu applizieren sind.

EP-A-1116700 offenbart eine Glaszusammensetzung, die kein $SiO_2$ umfasst und des Weiteren sind keine Materialien genannt, die auf die Haut aufzutragen oder zu applizieren sind.

Allen zuvor beschriebenen aus dem Stand der Technik bekannten antimikrobiell wirksamen Verbandsmaterialien, die Silber enthalten, ist jedoch zumindest ein Nachteil inne. Die silberhaltigen Verbandsmaterialien zeigen nach einer gewissen Zeit aufgrund der Bildung elementaren Silbers bzw. Silberoxids eine Dunkelfärbung. Vor allem durch Wärme, Feuchtigkeit, Licht und/oder Strahlungseinflüssen wird die Dunkelfärbung beschleunigt. Das unästhetische dunkelbraune oder schwarze Silberoxid hat zudem keinerlei keimabtötende Wirkung mehr, so dass die Dauerhaftigkeit der Wirksamkeit der bekannten Verbandsmaterialien leidet. Damit lässt die antimikrobielle Wirksamkeit nach kurzer Zeit nach oder aufwendige Versiegelungs- und Verpackungsschritte sind für derartige Produkte notwendig. Insbesondere genügen diese Produkte nicht den ästhetischen Ansprüchen der Konsumenten.

Es bestand daher der Wunsch nach einem antimikrobiellen Polymermaterial, das als Verbandsmaterial, insbesondere als Wundauflage, angewendet alle positiven Eigenschaften der einzelnen bekannten antimikrobiellen Verbandsmaterialien in sich vereint und gleichzeitig die bestehenden Nachteile dieser Materialien vermeidet. Insbesondere soll das Polymermaterial

- bevorzugt selbstklebend ausgerüstet sein, um zusätzliche Befestigungsmaterialien zu ersparen,
- einfach im Aufbau, um unkompliziert auch von Laien handhabungsfähig zu sein,
- ggf. ein feuchtes Wundmilieu generieren,
- antimikrobielle wirksam sein, und das auch mit möglichst geringen Wirkstoffanteilen,
- lagerfähig sein, ohne Einbussen hinsichtlich zuvor genannter Eigenschaften,
- ästhetischen Ansprüchen der Konsumenten genügen,
- stabil gegen äußere Einflüsse, wie Licht, Feuchtigkeit und/oder Strahlung

Insbesondere soll das antimikrobiellen Polymermaterial den ästhetischen Ansprüchen genügen und sich auch nach längerer Zeit nicht verfärben.

Diese Bündel an Aufgaben galt es zu lösen.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt. Dem effektiven Schutz, auch gerade der verletzten oder anderweitig behandelten menschlichen Haut vor Eindringen von Bakterien kommt daher eine wichtige Rolle zu.
Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung ist es, den Stand der Technik in dieser Richtung zu bereichern, insbesondere also, kosmetische Zubereitungen zur Verfügung zu stellen, welche antimirkobiell und/oder desinfizierend

wirksam sind, ohne dass mit der Anwendung der Substanzen eine unvertretbare Beeinträchtigung der Gesundheit des Anwenders verbunden wäre. Desinfizierend und/oder antimikrobiell wirkende Zubereitungen sind beispielsweise aus der Aknebehandlung bekannt. Die ACNE CREME ist eine leicht desinfizierende Creme. Sie eignet sich für jene Fälle von Akne, die eine Schälbehandlung erfordern. Weitere bekannte kosmetische Zubereitungen, die desinfizierend und entzündungshemmend wirkten, enthalten Wirkstoffe, wie beispielsweise Chlorhexidin, Chlorhexidin-Gluconat, Hamamelis-Zinkoxid, Panthenol, Dexpanthenol und/oder Urea pur.

Nachteil dieser Kosmetika allerdings sind teilweise Reizungen der Haut (Rötung, Schuppung) sowie in manchen Fällen allergische Reaktionen.

Aufgabe der vorliegenden Erfindung ist es daher auch kosmetische Zubereitungen bereit zu stellen, die eine Linderung bei gereizten Hautzuständen bzw. die Unterstützung der Wiederherstellung der Hauthomöostase mit einer gleichzeitigen Pflege der Haut verbinden.

Zur kosmetischen Behandlung der Haut und der Haare zählt auch deren Reinigung. Reinigung bedeutet sowohl Entfernung unerwünschten Schmutzes, Beseitigung von Keimen aller Art als auch Erhöhung des psychischen und physischen Wohlbefindens. Für die Reinigung kommen in erster Linie tensidhaltige Zubereitungen in Frage.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielsweise -COO', $-OSO_3^{2-}$, $-SO_3^-$, während die hydrophoben Teile in der Regel unpolare Kohlenwasserstoffreste darstellen.

Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und

nichtionische Tenside.

Antibakterielle Reinigungszubereitungen, wie z.B. Seifen, enthalten häufig Triclocarban (TCC), Triclosan (Irgasan DP 300) oder Chloroxylenol (PCMX). Diese Wirkstoffe blockieren ein bestimmtes Enzym, welches eine Reihe von Bakterien zum Überleben braucht. Durch die starke Verbreitung dieser Wirkstoffe kommt es jedoch zunehmend zu einer Resitenzentwicklungwirkung des Anwenders.

Aufgabe der vorliegenden Erfindung ist es daher auch eine Reinigungszubereitung bereit zu stellen, die antimikrobiell, und/oder desinfizierend sowie hautberuhigend und pflegend wirkt.

Gelöst werden diese Aufgaben durch ein Material, wie es im Hauptanspruch niedergelegt ist. Gegenstand der Unteransprüche sind dabei vorteilhafte Weiterbildungen des Materials und deren Verwendung.

Es war überraschend und für den Fachmann nicht vorauszusehen, dass ein Material, das geeignet ist zum Auftragen oder Applizieren auf die Haut, enthaltend antimikrobiell wirksames silberhaltiges Glas der Zusammensetzung

| | |
|---|---|
| $P_2O5$ | 40 - 60 mol%, |
| $R^1O$ | 35 - 55 mol%. |
| $R^2{}_2O$ | 0 - 5 mol%. |
| $SiO_2$, $Al_2O_3$ | 5 - 20 mol%, bezogen auf die Gesamtmenge des silberoxidfreien Glases, und |
| $Ag_2O$ | 0,1 bis 5 Gew.%, bezogen auf die Gesamtmasse des Glases |

wobei $R^1$ gewählt wird aus Ca, Mg, Zn und/oder Cu und $R^2$ gewählt wird aus Na. K und/oder Li und wobei das Material gewählt ist

e.) aus der Gruppe der Polymermaterialien Polyacrylate, Polyisobutylen, Styrolblockcopolymere (SBC), SIBS-Massen, SEBS-Massen, Silikone. Kautschukmassen, Chitosane, Alginate, Hydrogele, Hydrokolloide, Gelmatrices auf Agar Agar/PAS-Basis, Polymeren auf PVA/PAS-Basis und/oder Polyurethane,

f.) aus der Gruppe der kosmetischen Zubereitungen, Emulsionen, insbesondere in Form von W/O-, O/W-, W/OAN- oder O/W/O-Emulsionen, Mikroemulsion, Pickerina-Emulsion oder sprühbare Emulsion sowie wässrige Hydrogele.

g.) aus der Gruppe der Tücher, Pads, Hautauflagen, wobei die Materialien mit einer das Silberglas enthaltenden Tränkungslösung befeuchtet sind, oder

h.) aus der Gruppe der Reinigungszubereitungen, insbesondere Schaum- und Duschbäder, feste und flüssige Seifen

oder so genannte "Syndets" (synthetische Detergentien). Shampoos, Handwaschpasten, Intimwaschmittel, spezielle Reinigungsmittel für Kleinkinder, Duschgele, Cleansing-Zubereitung, Make-up-Entferner oder Rasierprodukte,

das Bündel an Aufgaben vollständig löst.

Die Angabe der Glaszusammensetzung in mol% gibt die Bestandteile ohne Silberoxid an. Zusätzlich ist die Silberoxidmenge in Gew.%, bezogen auf die Gesamtmasse des dann silberhaltigen Glases angegeben.

Besonders ein Material mit einer Glaszusammensetzung

| | |
|---|---|
| $P_2O_5$ | 45 - 55 mol%, |
| CaO, MgO | 35 - 50 mol%, |
| $Na_2O$, $K_2O$ | 0- 5 mol%, |
| $SiO_2$ | 0-5 mol%. |
| $Al_2O_3$ | 5 - 15 mol% und |
| $Ag_2O$ | 0,5 - 3 Gew.% sowie bevorzugt ein Material mit einer Glaszusammensetzung |
| $P_2O_5$ | 50 mol%, |
| MgO | 44 mol%, |
| $Al_2O_3$ und | 6 mol%, bezogen auf die Gesamtmenge des silberoxidfreien Glases |
| $Ag_2O$ | 2 Gew.%, bezogen auf die Gesamtmasse des Glases, oder mit einer Glaszusammensetzung |
| $P_2O_5$ | 73,35 Gew.%, |
| MgO | 18,33 Gew.%, |
| $Al_2O_3$ | 6,32 Gew.% und |
| $Ag_2O$ | 2,0 Gew.%, bezogen auf die Glasgesamtmasse, hat sich als besonders anwendungsfreundlich und wirksam erwiesen. |

Diese Materialien sind insbesondere Polymermaterialien, wie beispielsweise Polyurethane, Kosmetika, insbesondere kosmetische Zubereitungen auf Emulsionsbasis, desinfizierende Reinigungszubereitungen sowie antimikrobiell wirksame Hautauflagen, Pads oder Tücher.

Diese Materialien enthalten bevorzugt 0,001 bis 40 Gew.%, bevorzugt 0,05 bis 1 Gew.%, des silberhaltigen Glases, bezogen auf die Gesamtmasse des Materials.

Das hierbei enthaltende antimikrobiell oder desinfizierend wirksame Silber ist in Form freier Silberionen verfügbar und nur der Zusammensetzungsschreibweise des Glases entsprechend als Oxid $Ag_2O$ gekennzeichnet.

Eine bevorzugte Ausführungsform der erfindungsgemäßen Materialien umfasst Polymermaterialien. Das Polymermaterial dient dabei in erster Linie als Verbandsmaterial zum Auftragen auf die menschliche Haut. Unter Verbandsmaterialien werden auch Wundauflagen verstanden, so dass insbesondere das erfindungsgemäße Polymermaterial vorteilhaft als Wundauflage einzusetzen ist.

Gegenstand der Erfindung ist somit auch ein Polymermaterial mit antimikrobiellen Eigenschaften, dadurch gekennzeichnet, dass Materialien, die in der Wundheilung Verwendung finden, wie synthetische Polymermaterialien, zum Beispiel Polyurethane, Polyacrylate, SIBS-Massen, SEBS-Massen, Naturkautschukmassen sowie Chitosane, Alginate, Hydrogele, Hydrokolloide, insbesondere aber Polyurethane, kombiniert werden mit den silberhaltigen Gläsern, die in bevorzugten Ausführungsformen der Erfindung mit 0,01 bis 40 Gew.%, insbesondere bevorzugt 0,05 bis 1 Gew. %, in die Polymermaterialien eingearbeitet werden können. Dabei ist es nicht erforderlich, dass die Matrix zwingend geschäumt sein muß, wie es im Stand der Technik zur effektiven Silberfreisetzung gefordert wird.

Bevorzugt werden selbstklebende Polymermaterialien ausgewählt, um eine zusätzliche Randverklebung der Wundauflage zu vermeiden.

Erfindungsgemäß besonders hervorzuheben ist die Verwendung der silberhaltigen Gläser als Bestandteil in einer selbstklebend ausgerüsteten Polyurethanmatrix, die als hydroaktive Wundauflage für die feuchte Wundheilung eingesetzt werden kann.

Vorzugsweise kommen elastische, vernetzte Polyurethane mit einem Masseauftragsgewicht von 50 bis 2500 $g/m^2$ zum Einsatz, wie sie zum Beispiel in der WO 97/43328 A1 beschrieben sind.

In der Regel werden Polyurethane aus den bekannten Ausgangsverbindungen der Polyurethanchemie nach

bekannten Verfahren hergestellt, die in den Patenten DE-OS 3103499, DE-OS 3103500, EP 0 147 588 A1, EP 0 665 856 B1 oder DE 196 18 825 A1 dargestellt werden.
Polyurethan wird als Grundlage für das Polymermaterial verwendet. Die Herstellung des Polyurethans (c) erfolgt durch die Polymerisation eines Alkohols (a) mit einem Isocyanat (b).

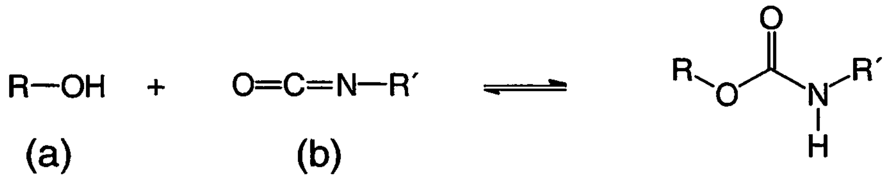

(a) (b) (c)

Ein entscheidender Vorteil der Polyurethanpolymer- oder gelmatrices sind ihre selbstklebende Eigenschaften, die ein zusätzliches Aufbringen einer Adhäsionsschicht auf die Matrix, zur Fixierung des Polymermaterials im Bereich der Haut, überflüssig machen. Im einfachsten Fall befindet sich die silberhaltige Polyurethanmatrix zwischen einer mit ihr fest verankerten Abdeckschicht, auch als Trägerschicht benannt, und einer abziehbaren Trennschicht.
Die abziehbare Trennschicht dient zur Sicherung der Klebeschicht, zur Verbesserung der Transport- und Lagerstabilität und wird vor dem Applizieren auf die Haut entfernt.
Geeignete Polyurethane als Matrix sind Gegenstand der DE 196 18 825, in der hydrophile, selbstklebende Polyurethangele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von 10 Gew.%,
b) Antioxidantien,
c) in den Polyolen a) löslichen Wismut-(III)-Carboxylaten auf Basis von Carbonsäuren mit 2 bis 18 C-Atomen als Katalysatoren sowie
d) Hexamethylendiisocyanat, mit einem Produkt der Funktionalitäten der Polyurethan-bildenden Komponenten a) und d) von mindestens 5,2, wobei die Isatalysatormenge c) 0,005 bis 0,25 Gew.%, bezogen auf das Polyol a) beträgt, die Menge an Antioxidantien b) im Bereich von 0,1 bis 1,0 Gew.%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥ 20 Gew.%.
Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.
Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.% liegt.
Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)Salze verzweigter gesättigter Carbonsäuren mit

tertiären Carboxylgruppen, wie der 2,2-DimethylOctansäure (zum Beispiel Versatic-Säuren, Shell). Gut geeignet sind Zubereitungen dieser Bi(III)Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuss von 3 mol dieser Säure mit einem Bi-Gehalt von ca. 17%.

Es werden die Katalysatoren bevorzugt in Mengen von 0,03 bis 0,1 Gew.%, bezogen auf das Polyol a), eingesetzt.

Als Antioxidantien kommen für die erfindungsgemäßen Polyurethangele insbesondere sterisch gehinderte phenolische Stabilisatoren, wie BHT (2,6-Di-tert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4-hydroxyphenyl)-propionat) (Ciba-Geigy) oder Tocopherol (Vitamin E) in Betracht. Bevorzugt werden solche vom Typ des α-Tocopherol eingesetzt. Die Antioxidantien werden bevorzugt in Mengen von 0,15 bis 0,5 Gew.%, bezogen auf das Polyol a), eingesetzt.

Die Isocyanatkennzahl (Verhältnis der bei der Reaktion eingesetzten freien NCO-Gruppen zu den freien OH-Gruppen) der erfindungsgemäßen Polyurethangelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$f_{(Polyol)} \circ (f_{(Isocyanat)} - 1) \circ Kennzahl \approx 2$$

$$Kennzahl \approx \frac{2}{f_{(Polyol)} \circ (f_{(Isocyanat)} - 1)}$$

f: Funktionalität der Isocyanat- oder Polyolkomponente

Je nach angestrebter Klebrigkeit der Elastizität des Gels kann die tatsächlich zu verwendende Isocyanatkennzahl um bis zu ± 20% von dem berechneten Wert abweichen.
Die erfindungsgemäßen Polyurethangelmassen werden hergestellt nach üblichen Verfahren, wie sie beispielsweise beschrieben sind in Becker/Braun, Kunststoff-Handbuch, Bd. 7, Polyurethane, S. 121 ff, Carl-Hauser, 1983.

Weiter vorzugsweise kommen Polyurethangele zum Einsatz, wie sie in der EP 0 665 856 B1 offenbart sind. Die hydrophilen Polyurethane sind demnach erhältlich aus

1. einem Polyurethangel, welches

   (A) 25-62 Gew.%, vorzugsweise 30-60 Gew.%, besonders bevorzugt 40-57 Gew.%, bezogen auf die Summe aus (A) und (B), eines kovalent vernetzten Polyurethans als hochmolekulare Matrix und
   (B) 75-38 Gew.%, vorzugsweise 70-40 Gew.%, besonders bevorzugt 60-43 Gew.%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls
   (C) 0 bis 100 Gew.%, bezogen auf die Summe aus (A) und (B), an Füll- und/oder Zusatzstoffen enthält,

   und welches erhältlich ist durch Umsetzung einer Mischung von

   a) einem oder mehreren Polyisocyanaten,
   b) einer oder mehreren Polyhydroxylverbindungen mit einem mittleren Molekulargewicht zwischen 1000 und 12000, und einer mittleren OH-Zahl zwischen 20 und 112,
   c) gegebenenfalls Katalysatoren oder Beschleunigern für die Reaktion zwischen Isocyanat- und Hydroxylgruppen sowie gegebenenfalls
   d) aus der Polyurethanchemie an sich bekannten Füll- und Zusatzstoffen, wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen 3

und 6 beträgt und die Isocyanatkennzahl (K) der Formel

$$K = \frac{300 \pm X}{(F_l \bullet F_p) - 1} + 7$$

gehorcht, in welcher X ≤ 120, vorzugsweise X ≤ 100, besonders bevorzugt X ≤ 90 ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierenden Material und/oder
3. einem nichtwässrigen Schäumungsmittel.

Bei der Herstellung von bevorzugt selbstklebenden Polyurethanen ist zu berücksichtigen, dass bei der Auswahl der gelbbildenden Komponenten die oben definierten Bedingungen eingehalten werden, da sonst anstelle von selbsthaftenden Gelen klebfreie, elastische Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisat bzw. hydrierte Diphenylmethandüsocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen.

Die Diisocyanate können insbesondere zum Beispiel aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Als Vorteile der erfindungsgemäßen Polyurethane im Vergleich zu anderen Polymeren, die für die Herstellung von Verbandsmaterialien verwendet werden, lassen sich folgende Punkte nennen:

- Polyurethan kann flexibel als selbstklebende oder nichtklebende Matrix bereit gestellt werden.
- als selbstklebendes System kann auf einen Zusatz weiterer Klebstoffe, die unter Umständen Nebenwirkungen wie Mazeration, Entzündungen der dermalen Bereiche, Reduktion der Hautatmung u.a. hervorrufen, verzichtet werden.
- Polyurethane erweisen sich gegenüber anderen Klebematerialen, wie Polyacrylate, Kautschuk etc., als äußerst vorteilhaft, da sie kein Allergiepotential beinhalten.
- Polyurethan weist eine sehr gute Wasserdampfdurchlässigkeit auf. Hierdurch ist gewährleistet, dass bei einer Applikation über einen längeren Zeitraum keine Mazeration durch die Wasserabgabe der Haut erfolgt.
- Die Sauerstoffdurchlässigkeit des Polyurethan sorgt für eine gute Versorgung der abdeckten Hautstelle mit Sauerstoff, wodurch einer Schädigung des Gewebes entgegengewirkt wird.
- Polyurethan ist allergieneutral, so dass nach der Applikation mit keiner allergischen Reaktionen des Organismus zu rechnen ist.
- Polyurethan zeigt zudem gegenüber anderen Materialien wie z.B. Hydrocolloiden oder Hydrogelen keine Neigung bei längerem Kontakt mit Flüssigkeiten wie Wundexudat zu desintegrieren. Ein aus Polyurethan hergestellter Wundverband hinterläßt demnach, bei längerem Kontakt mit Wundflüssigkeit, keine die weitere Wundheilung störenden Rückstände in der Wunde.
- Selbstklebend ausgerüstetes Polyurethan entklebt bei Kontakt mit Flüssigkeit, so dass ein Verkleben mit frisch gebildetem Gewebe vermieden wird und zudem eine schmerzfreie Ablösung des Wundabdeckung gewährleistet ist.
- Erfindungsgemäße Polyurethanwundauflagen erzeugen ein feuchtes Wundmilieu, was zu einer schnelleren Wundheilung führt.

Neben den bevorzugten Polyurethanmatrices lassen sich auch Polymermaterialien auf anderer Grundlage verwenden.

Das einzuarbeitende Silberglas welches die jeweilige grundlegende Vernetzungsreaktion der Polymermatrix beeinträchtigt, kann bedarfsweise nach der Vernetzungsreaktion oder der Entstehung der Matrix als Lösung oder in Form einer kosmetischen Emulsion in die Matrix eingebracht werden.

Besonders vorteilhafte selbstklebende silberglashaltige Matrices können aus folgenden lösemittelhaltigen oder lösemittelfreien, gefüllten oder nichtgefüllten, vernetzenden oder nichtvernetzenden, hydrophilen oder hydrophoben

Matrixsystemen hergestellt werden:

**Polyisobutylenmatrices**

Die haftklebrige Matrix zur kontrollierten Abgabe von Silberionen aus Silberglas an die Haut, wobei die haftklebrige Matrix frei von Mineralölen und Klebharzen ist und aufgebaut ist aus

a) synthetischen Gerüstpolymeren auf der Basis von Polyisobutylen zu 25 bis 90 Gew.%,
b) amorphem Poly-α-olefin zu 5 bis 30 Gew.%,
c) einem unlöslichen, insbesondere hydrophilen Füllstoff zu 0 bis 60 Gew.% und
d) Silberglas zu 0,005 bis 10 Gew.%.

In einer vorteilhaften Ausführungsform der Matrix setzt sich das Polyisobutylen zusammen aus hochmolekularem PIB zu 5 bis 55 Gew.% und niedermolekularem PIB zu 20 bis 60 Gew.%.
Eine typischer erfindungsgemäßer Haftklebstoff besteht somit aus folgenden Komponenten:

| | |
|---|---|
| hochmolekulares PIB | 5 - 55 Gew. %, bevorzugt 25 - 45 Gew. % |
| niedermolekulares PIB | 20 - 60 Gew.%, bevorzugt 30 - 50 Gew. % |
| amorphes Poly-α-olefin | 5 - 30 Gew.%, bevorzugt 5 -10 Gew. % |
| hydrophiler Füllstoff | 0 - 60 Gew.%, bevorzugt 0 - 30 Gew. % |
| Silberglas | 0,005 - 10 Gew.%, bevorzugt 0,01 - 5 Gew. % |

Optional können noch bis zu 20 Gew.% eines permeationsfördernden Hilfsstoffes (lipophiler Lösungsvermittler/Enhancer) wie Ölsäuredecylester, Isopropylmyristat und -palmitat (IPM und IPP) zugesetzt werden.
Die genannten Rezepturbestandteile werden dabei wie folgt genauer definiert: Hochmolekulares PIB bedeutet:

Polyisobutylen mit einem gewichtsmittleren Molekulargewicht ($M_w$) von 300.000 bis 1.100.000, bevorzugt zwischen 650.000 und 850.000. Solche Polymere sind kommerziell beispielsweise unter den Handelsnamen Oppanol B100 oder Vistanex MM-L80 erhältlich.

Niedrig molekulares PIB bedeutet:

Polyisobutylen mit einem gewichtsmittleren Molekulargewicht ($M_w$) von 40.000 bis 300.000, bevorzugt zwischen 60.000 und 100.000. Solche Polymere sind kommerziell beispielsweise unter den Handelsnamen Oppanol B15 oder Vistanex LMMH erhältlich.

Amorphes Poly-α-olefin bedeutet:

Amorphe Copolymere auf der Basis von Ethylen und Propylen, Butylen oder 1-Hexen. Das bevorzugte gewichtsmittlere Molekulargewicht ($M_w$) liegt bei 5.000 bis 100.000, bevorzugt zwischen 10.000 und 30.000. Solche Polymere sind kommerziell beispielsweise unter den Handelsnamen Eastoflex ® oder Vestoplast ® erhältlich.

In der genannten Polymermatrix gelten unlösliche, hydrophile Partikel auf der Basis von Cellulose als hydrophile Füllstoffe. Bevorzugt ist eine mittlere Partikelgröße von kleiner gleich 100 μm mit einer möglichst gleichförmigen Oberfläche. Solche Materialien sind zum Beispiel unter den Handelsnamen Avicel und Elcema kommerziell erhältlich.
Bevorzugt erfolgt die Herstellung der PIB-Matrix in einem Verfahren, bei dem alle Komponenten der haftklebrigen Matrix unter Verzicht auf den Zusatz von Lösungsmittel in der Schmelze homogenisiert werden.
Besonders bevorzugt werden alle Komponenten in einem kontinuierlichen oder diskontinuierlichen Prozeß bei einer Temperatur unterhalb von 100 °C verarbeitet.
Weitere vorteilhafte Ausführungsformen der einsetzbaren Polyisobutylenmatrices können den Druckschriften DE 100 56 010 oder DE 100 56 011 entnommen werden, die hiermit ausdrücklich zum Offenbarungsgehalt der vorliegenden Erfindung zählen.

**Polyacrylsäurematrices**

Polyacrylate sind ebenfalls vorteilhaft im Sinne der erfindungsgemäßen Matrices. Erfindungsgemäß vorteilhafte Polyacrylate sind Acrylat-Alkylacrylat-Copolymere, insbesondere solche, die aus der Gruppe der so genannten Carbomere oder Carbopole (Carbopol® ist eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

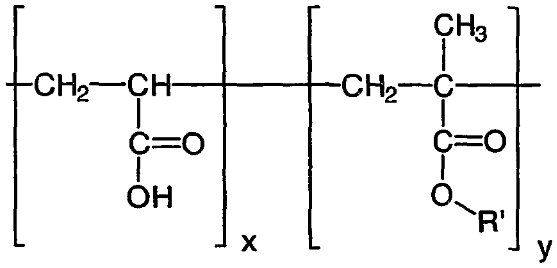

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001

Ferner vorteilhaft sind Copolymere aus $C_{10-30}$-Alkylcacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/$C_{10-30}$ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die Ammoniumacryloyldimethyltaurate/Vinylpyrrolidoncopolymere die Summenformel $[C_7H_{16}N_2SO_4]_n$ $[C_6H_9NO]_m$ auf, einer statistischen Struktur wie folgt entsprechend

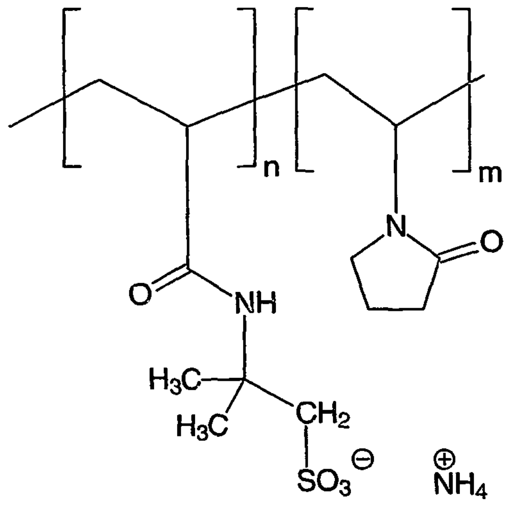

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Bei einer erfindungsgemäßen bevorzugten silberglashaltigen Polyacrylsäurematrix wird die Vernetzung der Polyacrylsäure mit Hilfe von Polyvinylpyrrolidon (PVP) durchgeführt.

Die Vernetzung läuft über die Bildung eines quartären Ammoniumsalzes des PVP. Diese Art der Vernetzung führt zu organischen Salzen, die im Gegensatz zu den bekannten Metallsalzen als Vernetzungsagentien über die Hydroxyfunktionen der Polyacrylsäuremoleküle gebunden werden. Wie bei den Metallsalzen ist die Reaktion reversibel und kann durch die Zugabe von Wasser oder Säuren umgekehrt werden. Die Viskosität des resultierenden Gels läßt sich nicht nur über die Menge an Vernetzer steuern, sondern auch über das Molekulargewicht des PVP's. Dabei führen hohe Molekulargewichte zu Gelen mit niedriger Viskosität und niedrige Molekulargewichte zu Gelen mit hoher Viskosität und Klebkraft. Der Vorteil bei dieser Art der Vernetzung liegt darin, über die Parameter Anteil-PVP und Molekulargewicht-PVP zielgerichtet Gelmatrices herzustellen, deren Klebrigkeit, Kohäsivität und Viskosität individuell auf den jeweiligen Anwendungsbereich einstellbar sind.
Dieser Effekt des Einflusses des Molekulargewichtes des PVP auf die Viskosität und Klebkraft der Gelmatrix ist auf folgende Erkenntnis zurückführen: Bei langkettigem PVP ist die Zahl an Pyrrolidon-Untereinheiten pro Makromolekül deutlich höher als bei kurzkettigem PVP. Dadurch kommt es vermehrt zu Reaktionen der gleichen Reaktionspartner untereinander, da die Makromoleküle sich leicht zu Bündeln orientieren können. Diese Reaktionen führen nicht zur Bildung von Verknüpfungspunkten mit mehreren Polyacrylsäuremolekülen. Es werden daher nur wenige Querverbindungen zu anderen Polyacrylsäuremolekülen und damit nur wenige, große Maschen geknüpft. Dieser Umstand führt zu einem locker verknüpften Gel mit niedriger Viskosität. Im Gegensatz dazu werden bei kurzkettigem PVP aufgrund der höheren Beweglichkeit und der niederigeren Tendenz zur Orientierung der Moleküle zu Strängen mehr Verknüpfungen zu verschiedenen Polyacrylsäuremolekülen gebildet, die zu einer engeren Maschenweite und einer geringeren Flexibilität und Viskosität des Gels führen.
Die Viskosität der Gele läßt sich darüber hinaus noch über andere Faktoren steuern. So spielt beispielsweise die Menge an PVP eine mit entscheidende Rolle für die Struktur des Gels. Wird ein Sättigungspunkt überschritten, so kommt es zu konkurrierenden Reaktionen der freien PVP Moleküle mit den bereits vernetzten. Diese Konkurrenzreaktionen führen dazu, dass Vernetzungspunkte zugunsten von nicht verknüpften Aggregaten aus Polyacrylsäure und den überschüssigen PVP Molekülen aufgebrochen werden. Die Folge dieser Übersättigung ist eine Abnahme der Gesamtzahl an Verknüpfungspunkten und damit eine Abnahme der Gelviskosität. Als weitere Möglichkeit zur Steuerung der Gelviskosität kann die Zugabe von protischen Lösungsmitteln (z.B. Wasser, Alkohole, Amine, Thiole) oder organischen Protonendonatoren (Carbonsäuren z.B. Salicylsäure) oder anorganischen Agentien (z.B. Lewis-Säuren) genutzt werden. Hier bieten sich speziell Verbindungen aus den Substanzklassen der tertiären Polyamine und der Polyamide an. In jedem dieser Fälle trägt die Zugabe der Agentien zur Verringerung der Koordinationsstellen entweder an der Polyacrylsäure oder am PVP bei. Dadurch wird die Zahl der potentiellen Verknüpfungspunkte zur Ausbildung von Gelmaschen verringert, was einen direkten Einfluss auf die Viskosität des Gels hat.

Des weiteren lassen sich die resultierenden Geleigenschaften der Matrices über das Molekulargewicht, Substitutions- und Vernetzungsgrad der eingesetzten Polyacrylsäure beeinflussen.
Zur Ausfertigung besonderer anwendungstechnischer Eigenschaften werden die Gelmatrices mit den entsprechenden Weichmachern, Lösungsvermittlern, Penetrationsenhancern, Füllstoffen und/oder anderen bekannten Zusätzen versetzt. Als Gelgrundlage vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die vorteilhaften Acrylat-Alkylacrylat-Copolymere durch die folgende Struktur aus:

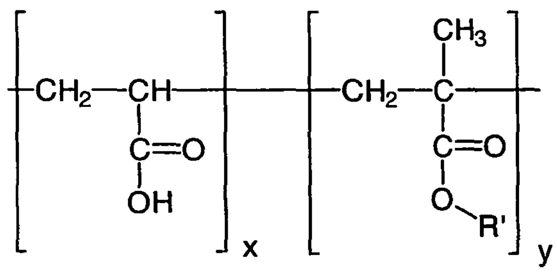

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Polyacrylsäure und/oder deren Copolymere werden bevorzugt in einer Menge von 5 - 55 Gew.%, besonders bevorzugt zwischen 5-30 Gew.% eingesetzt. Alle Prozentangaben beziehen sich dabei Gewichtsanteile Gelmatrix sofern nicht Gegenteiliges angegeben ist.

Als Vernetzer wird Polyvinylpyrrolidon (PVP), z.B. Luviskol der Firma BASF, eingesetzt, bevorzugt in einer Menge von 0,25 - 60 Gew.%, besonders bevorzugt zwischen 1-30 Gew.%. In gleichem Maße können auch_PVP-Copolymerisate wie beispielsweise Vinylpyrrolidon-Vinylacetat (Povidonacetat; Kollidon VA 64), Terpolymere auf Basis Vinylpyrrolidon und Acrylsäure oder Methacrylsäure bzw. deren Ester (Luviflex VBM 35), Copolymerisate aus Vinylpyrrolidon und Vinylimidazoliummethochlorid (Luviquat-Marken) als sog. PVP- Vemetzungsagenz eingesetzt werden.

Als weitere Gelbestandteile können Polyalkohol oder -alkohole, z.B. 1,2-Propandiol, Glycerin, und/oder Wasser eingesetzt werden, bevorzugt in einer Menge von 5 - 90 Gew.%, besonders bevorzugt zwischen 5 - 45 Gew.%.

Weitere Bestandteile der Gelmatrix können Lösungsvermittler, z.B. Polyethylenglycole (Lutrol E400, E600 der Firma BASF) in einer Menge von 0-50 Gew.%, bevorzugt 0-30 Gew.%, Neutralisationsmittel, z.B. Tromethamol, Triethanolamin und/oder Dexpanthenol, in einer Menge 0-30 Gew.%, bevorzugt 0 - 15 Gew.%, Füllstoff(e), z.B. Kieselsäure, mikronisierte Cellulose und/oder Gelatine, in einer Menge von 0-30 Gew.%, bevorzugt 3-15 Gew.%, und natürlich Wirkstoff(e), z.B. Menthol oder Jojobaöl , in einer Menge von 0-35 Gew.%, bevorzugt 0-15 Gew.%, sein.

Die Herstellung dieser silberglashaltigen Polyacrylsäurematrices erfolgt lösemittelfrei, vorzugsweise bei Raumtemperatur, in handelsüblichen Knetern oder geeigneten Extrudern.

Weitere vorteilhafte Ausführungsformen von Polyacrylsäurematrices zur Verwendung als silberglashaltige Matrix können der Patentanmeldung DE 101 42 918 entnommen werden, die hiermit ausdrücklich zum Offenbarungsgehalt der vorliegenden Erfindung zählt.

## Silikonmatrices

Die feuchtigkeitsaufnehmende silberglashaltige Matrix auf Silikonbasis zur kosmetischen oder pharmazeutischen Hautbehandlung mit einer haftklebrige Matrix besteht aus

a) Silikon
b) Gelbildner
c) gegebenenfalls einem Silikonharz.

In einer ersten vorteilhaften Ausführungsform der Erfindung weist die Matrix folgende Zusammensetzung auf:

| | | |
|---|---|---|
| a) Silikon: | 55 bis 80 Gew.%, | insbesondere 60 bis 75 Gew.% |
| b) Gelbildner: | 20 bis 40 Gew.%, | insbesondere 25 bis 40 Gew.% |
| c) Silberglas | 0,01 bis 10 Gew.%, | insbesondere 0,5 - 5 Gew. % |

Silikone werden als Ein- oder Zweikomponentensysteme verarbeitet. Die Vernetzung erfolgt dabei in der Regel als Polykondensation unter Abspaltung von Essigsäure, oder als Polyaddition unter Verwendung eines Platinkatalysators.

Für die Herstellung der beschriebenen Matrices wurde ein handelsübliches Zweikomponentensystem aus Polydimethylsiloxan (siehe Abbildung), und zwar Q7-9600 A+B; Fa. Dow Corning,

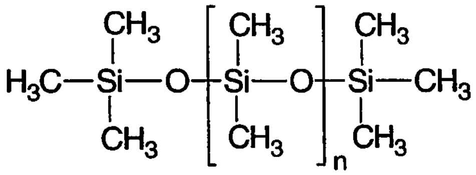

verwendet.

Zur Einstellung der Klebkraft wurde optional ein mit Silikonharz quervernetztes Polydimethylsiloxan (PSA MD74602; Fa. Dow Coming) verwendet.

Eine Wasseraufnahmefähigkeit der Matrix wurde erreicht, indem man Gelbildner mit hoher relativer Oberfläche in solchen Mengen einarbeitet, dass der Gelbildner intermolekulare Querverbindungen von der Oberfläche zum Inneren der Matrix haben kann. Solche Gelbildner sind zum Beispiel Polyacrylsäure, Polyacrylnitril oder mikrokristalline Cellulose. Hauptsächlich wurden Polyacrylsäuretypen der Carbopol-Reihe, Goodrich Corp., eingesetzt.
Zur Variation der Wasseraufnahmefähigkeit wurden zusätzlich starke Gelbildner mit niedriger relativer Oberfläche eingearbeitet, wie zum Beispiel Natrium-Polyacrylat (Favorsorb; Fa. Stockhausen).
Die Herstellung erfolgt bei Raumtemperatur in handelsüblichen Mischern. Zunächst werden bei 2-Komponentensystemen die beiden Silikonkomponenten miteinander vermischt. Danach wird bedarfsweise die Silikonharzkomponente eingerührt, dann der beziehungsweise die Gelbildner eingearbeitet und abschließend Silberglas eingerührt.
Die Matrix wird auf einem Träger ausgestrichen und man läßt das Lösemittel der Silikonharzkomponente aus der Matrix verdunsten. Die Dauer der Vernetzungsreaktion der Silikonmatrix kann temperaturabhängig gesteuert werden. Die klebende Seite der Matrix wird mit einem Trennträger abgedeckt.
Optional kann das einzuarbeitende Silberglas auch nach der Vernetzungsreaktion als Lösung oder in einer kosmetischen Emulsion über die Kanäle der Gelbildner eingebracht werden.
Weitere vorteilhafte Ausführungsformen von Silikon-Matrices zur Verwendung als silberglashaltige Matrix können der Patentanmeldung DE 101 14 382 entnommen werden, die hiermit ausdrücklich zum Offenbarungsgehalt der vorliegenden Erfindung zählt.

**Kautschukmatrices**

Die feuchtigkeitsaufnehmende silberglashaltige Matrix auf Kautschukbasis zur kosmetischen oder pharmazeutischen Hautbehandlung, mit einer haftklebrigen Matrix, besteht aus

a) Kautschuk
b) Klebharzen
c) hydrophiler, wasseraufnehmender, dispers verteilter Feststoff.
d) Alterungsschutzsystem

In einer vorteilhaften Ausführungsform der Erfindung weist diese Matrix folgende Zusammensetzung auf:

| | |
|---|---|
| Silberglas 0,1 bis 10 Gew.%, | insbesondere 0,5 - 5 Gew.% |
| a) Kautschuk 20 -70 Gew.%, | insbesondere 30 - 50 Gew.% |
| b) Klebharz 10 - 50 Gew-%, | insbesondere 20 - 40 Gew.% |
| c) Füllstoff 10 -30 Gew-%, | insbesondere 12 - 20 Gew.% |
| d) Alterungsschutzsystem 0,5 -5 % | insbesondere 1 - 3 Gew.% |

Kautschukklebemassen in der angegebenen Form werden in Lösung verarbeitet. Der eingesetzte Kautschuk ist entweder natürlicher oder synthetischer Herkunft. Die mechanischen Eigenschaften der verschiedenen Typen ergeben im Gemisch einer Rezeptur die gewünschten Gerüsteigenschaften einer Kautschukklebemasse.
Die eingesetzten Klebharze vermitteln die notwendige Haftklebrigkeit oder Tack. Weichmacher, wie z.B. Mineralöle dienen zur Feinabstimmung der mechanischen Eigenschaften der Rezeptur.
Eine Wasseraufnahmefähigkeit der Matrix wurde erreicht, indem man eine hydrophilen wasseraufnehmenden Feststoff fein dispers in der Rezeptur verteilt.
Als Stoffgruppe kommen bevorzugt Pflanzenwurzel- und Stärkemehle zum Einsatz, möglich sind auch Cellulosen und deren Derivate sowie andere wasseraufnehmende Feststoffe. Die Wasseraufnahmefähigkeit kann über die Art und Menge des hydrophilen Feststoffes beeinflußt werden.
Kautschukklebemassen sind bedingt durch ihre Chemie oxidationsempfindlich. Sie müssen daher mit einem geeigneten Alterungsschutzsystem ausgerüstet werden. Dieses hat neben der Funktion als Antioxidans zusätzlich physiologisch unbedenklich zu sein.
Die Herstellung erfolgt bei Raumtemperatur in handelsüblichen Mischern. Die Kautschukbestandteile werden in einem geeigneten Lösungsmittel gelöst und anschließend werden die restlichen Komponenten inklusive des Wirkstoffes hinzugefügt.
Die Masse wird auf einem Träger ausgestrichen und man läßt das Lösemittel aus der Masse verdunsten. Die Dauer der des Verdunstungsvorgangs kann temperaturabhängig gesteuert werden. Die klebende Seite der Masse wird mit einem Trennträger abgedeckt.

**SBC-Hotmelt Matrices**

Bei der haftklebrigen Matrix zur kontrollierten Abgabe von Silberglas an die Haut, wird die gerüstbildnende Substanz der haftklebrigen Matrix durch Styrolblockcopolymere (SBC) gebildet.

In einer vorteilhaften Ausführung weist die latexfreie Haftklebemasse die nachfolgend angegebene Zusammensetzung auf:

| | |
|---|---|
| 5 Gew.% bis 90 Gew.% | Blockcopolymere, |
| 5 Gew.% bis 80 Gew.% | Klebrigmacher wie Öle, Wachse, Harze und/oder deren Mischungen, dabei bevorzugt Mischungen aus Harzen und Ölen, |
| weniger als 60 Gew.% | Weichmacher, |
| weniger als 15 Gew.% | Additive, |
| weniger als 5 Gew.% | Stabilisatoren |
| weniger als 10 Gew.% | silberglashaltige Zusätze. |

Die als Klebrigmacher dienenden aliphatischen oder aromatischen Öle, Wachse und Harze sind bevorzugt Kohlenwasserstofföle, -wachse und -harze, wobei sich die Öle (wie Paraffinkohlenwasserstofföle) oder die Wachse (wie Paraffinkohlenwasserstoffwachse) durch ihre Konsistenz günstig auf die Haftwirkung auswirken. In einer speziellen Form beinhaltet die Haftmasse mindestens ein aliphatisches Kohlenwasserstoffharz und mindestens ein aromatisches Kohlenwasserstoffharz. Als Weichmacher finden mit-tel- oder langkettige Fettsäuren und/oder deren Ester Verwendung. Diese Zusätze dienen dabei der Einstellung der Hafteigenschaften und der Stabilität. Gegebenenfalls kommen weitere Stabilisatoren und andere Hilfsstoffe zum Einsatz.

Ein Füllen der kohäsiven Haftmasse mit mineralischen Füllstoffen, Fasern, Mikrohohl- oder -vollkugeln ist möglich.

Optional können zusätzlich auch noch bis zu 15 Gew.% eines permeationsfördernden Hilfsstoffes als lipophiler Lösungsvermittler wie zum Beispiel Ülsäuredecylester oder sopropylmyristat und -palmitat (IPM und IPP) zugesetzt werden. ' Die Herstellung der Matrix erfolgt bevorzugt mittels Verfahren, bei denen alle Kompo-nenten der haftklebrigen Matrix unter Verzicht auf den Zusatz von Lösungsmitteln in der Schmelze homogenisiert werden.
Besonders bevorzugt werden dabei alle Komponenten in einem kontinuierlichen oder diskontinuierlichen Prozeß bei einer Temperatur unterhalb von 100 °C verarbeitet.

**Feuchtklebende Polymerfolie auf PVA/PAS-Basis**

Die feucht selbstklebende Polymerfolie zur desinfizierenden Abgabe von Silberionen aus Silberglas umfasst eine homogene Mischung aus Polyvinylalkohol mit einem durchschnittlichen Molgewicht von 20 000 bis 100 000 g/mol, bevorzugt 28 000 bis 40 000 g/mol, und einem Hydrolysegrad von 80 bis 95%, bevorzugt 85 bis 90%, und Polyacrylsäure mit einem durchschnittlichen Molgewicht von 450 000 bis 4 000 000 g/mol, bevorzugt 1 300 000 bis 4 000 000 g/mol, wobei das Gewichtsverhältnis Polyvinylalkohol zu Polyacrylsäure in der Polymerfolie im Bereich 10:1 bis 1:1 liegt.
In einer vorteilhaften Ausführungsform enthält die feucht selbstklebende Polymerfolie 0,001 -10 % Silberglas.

Dieser erfindungsgemäße Wundverband stellt ein lösemittelfreies Matrixsystem aus den synthetischen Polymeren Polyvinylalkohol und Polyacrylsäure dar, welches nur auf dem feuchten Wundgebiet haftet, das sich nach Verheilen der Wunde selbstständig und schmerzfrei wieder ablöst und mit einfachen technischen Mitteln kostengünstig herstellbar ist. Weiterhin ist der Wundverband luft- und wasserdampfdurchlässig.

Als besonders geeignet haben sich Wundverbände gezeigt bei denen das Gewichtsverhältnis Polyvinylalkohol zu Polyacrylsäure in der Folie im Bereich 5:1 bis 3:1 liegt.

Die feucht selbstklebende Polymerfolie kann weiterhin mit anderen Wirksubstanzen zur Wundbehandlung ausgerüstet werden. Der Wundverband kann zur besseren Wundheilung daher mit pharmazeutischen und/oder kosmetischen Wirkstoffen zur kontinuierlichen Freisetzung in die Wunde dotiert sein. Als bevorzugte pharmazeutische Wirkstoffe seien Dexpanthenol, Lidocain und Harnstoff genannt, als bevorzugte kosmetische Wirkstoffe seien beispielsweise die Gruppen der Flavonoide und Sericoside genannt. Der Anteil an Wirkstoffen im Wundverband beträgt vorteilhafterweise 0,01 bis 10 Gew.%. Der Wirkstoff wird während der Polymerbildung nach bekannten Verfahren in die Polymermatrix eingeführt.

In der einfachsten Ausführungsform kann der Wundverband als dünne Folie zur physikalischen Abdeckung und/oder zum Infektionsschutz auf oberflächliche feuchte Wunden appliziert werden. Aufgrund der Transparenz der Folie kann dabei der Wundheilungsverlauf über die Zeit ohne Entfernung der Wundabdeckung beobachtet werden ohne dass wie bei herkömmlichen Wundverbänden ein schmerzintensives Wiederablösen des Verbandes notwendig ist und ein neuer Wundverband aufgebracht werden muss.

Zur Erhöhung der inneren Festigkeit des Wundverbandes kann die Polymerfolie ein- oder zweiseitig geprägt sein. Ebenso kann durch ein eingelegtes Gitternetz aus synthetischem oder natürlichem Gewebe die Polymerfolie verstärkt ausgeführt sein. Als geeignete Gewebe kommen beispielsweise Baumwollnetzgewebe mit einer Maschenweite von 1 bis 2 mm oder ein gelochtes Vlies aus 67% Viskose und 33% Polyester in Frage.

In einer weiteren Ausführungsform wird die Polymermatrix einseitig mit einem Trägermaterial (Gewebe, Vliese, Schäume, Kunststoffe etc.) abgedeckt und als Verbundfolie appliziert. Je nach verwendetem Trägermaterial können dadurch die Wasserdampfdurchlässigkeit, die Festigkeit der Wundabdeckung, die Polsterung gegen Druck und andere physikalische Eigenschaften der Wundabdeckung gesteuert werden.

Der mit entscheidende Vorteil der Polymerfolie ist ihre auf feuchtem Grund selbstklebende Eigenschaft, die ein zusätzliches Aufbringen einer Adhäsionsschicht auf die Matrix, zur Fixierung des Wundverbandes im Bereich der Haut, überflüssig macht. Dies führt u.a. zu einer preiswerten und einfachen Herstellungsweise.

Die besondere Eigenschaft der feuchten Klebeeigenschaft des erfindungsgemäßen Wundverbandes erklärt sich aus der feinverteilten Einbindung der hydrophilen Polyacrylsäure in ein nur mäßig hydrophiles Polyvinylalkoholnetzwerk. Erfindungsgemäße Polyacrylate haben aufgrund ihrer Carboxylatfunktion eine starke Ladungsaffinität zu Wasser. Bei oberflächlichen Verletzungen der Haut ist diese für die Klebeigenschaften notwendige Feuchtigkeit in Form von Exudat und Blut vorhanden und der Wundverband saugt sich unter Gelbildung somit an der Wunde fest. Die wesentlich geringere Hydrophilie des Polyviniylalkohols sorgt dabei für eine gleichmäßige Verteilung der Feuchtigkeit in der Polymermatrix. Durch das mangelhafte Gelbildungsvermögen des Polyvinylalkohols bleibt jedoch die Festigkeit der Matrix dabei erhalten. Sobald aus der Wunde aufgrund von fortschreitender Verheilung keine ausreichende Feuchtigkeit mehr nachgeliefert wird, trocknet der Anteil an Polyacrylsäuregel aus und die Matrix verliert damit ihr Haftvermögen.

Erfindungsgemäß vorteilhafte Polyacrylate sind Acrylat-Alkylacrylat-Copolymere, wie unter der Rubrik Polyacrylsäurematrices beschrieben.

Die Erfindung umfasst ebenfalls herkömmliche Wundauflagen, wobei diese oberflächlich mit der erfindungsgemäß beschriebenen Polymerfolie ausgerüstet werden. Das Klebe-/Entklebungsverhalten der Wundauflagen handelsüblicher Pflaster kann damit gesteuert werden. Zur Herstellung wird eine entsprechende handelsübliche Wundauflage mit der noch wasserhaltigenhaltigen Polymermischung getränkt und diese erst anschließend getrocknet.

Zur Herstellung der feucht klebenden Folie wird ein filmbildendes Polymer hoher Kohäsivität wie z.B. Polyvinylalkohol (Mowiol 18/88; Fa.Hoechst) mit einem durchschnittlichen Molgewicht von 20 000 bis 100 000 g/mol, bevorzugt 28 000 bis 40 000 g/mol, und einem Hydrolysegrad von 80 bis 95%, bevorzugt 85 bis 90%, mit einem gelbildenden Polyacrylsäurepolymer, wie z.B. Carbopol 980 der Firma Goodrich, mit einem durchschnittlichen Molgewicht von 450 000 - 4 000 000 g/mol, bevorzugt 1 300 000 bis 4 000 000 g/mol wobei das Gewichtsverhältnis Polyvinylalkohol zu Polyacrylsäure im Bereich 10:1 bis 1:1 liegt, kombiniert. Beide Polymere werden in Wasser als Lösemittel bei 60 bis 90°C in einem zwangsdurchmischenden Rührwerk, wie z.B. einem Kneter, gelöst bzw. gequollen und homogen miteinander vermengt. Anschließend wird die entstandene viskose Masse flächig verstrichen und dann zur Folie getrocknet.
Zur Veränderung der physikalischen Eigenschaften der Folie wie z.B. der Elastizität können während des Knetvorgangs entsprechende Zusätze wie Polyethylenglykol (Lutrol E400; Fa.BASF) usw. eingearbeitet werden.
Als pharmazeutische/kosmetische Wirkstoffe können zusätzlich wundheilungsfördemde Agenzien z.B. Dexpanthenol und/oder andere Substanzen in die Matrix eingearbeitet werden.
Thermisch empfindliche Wirkstoffe, welche eine Einarbeitung in die Matrix bei 60 bis 90°C nicht erlauben lassen, können eingearbeitet werden, indem man die Matrix zunächst ohne Wirkstoff herstellt und trocknet. Diese temperaturempfindlichen Wirkstoffe können anschließend in einem hydrophilen Medium gelöst, durch die Matrix aufgesaugt werden. Danach wird die Matrix durch Verdunstung des Lösemittels bei Raumtemperatur oder in einer Gefriertrocknungsanlage erneut getrocknet.
Zur Einstellung eines besonders hautfreundlichen pH-Wertes des feucht klebenden Polymerfilms, und/oder zur Beeinflussung der Geleigenschaften der feuchten Polyacrylsäure können ebenfalls entsprechende pH-Wert-Korrigenzien wie Trometamol, Triethanolamin etc. in die Matrix eingearbeitet werden.

Optional zur Herstellung im Kneter kann die Polymerfolie auch kontinuierlich in einem Extruder hergestellt werden.

## Hydrokolloide

Die Gruppe der kosmetisch und pharmazeutisch relevanten Hydrokolloide lässt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie zum Beispiel Carboxymethylcellulose und andere Celluloseether, Hydroxyethyl- und -propylcellulose und mikrokristalline Cellulose dergleichen,
- organische, vollsynthetische Verbindungen, wie zum Beispiel Polyacryl- und Polymethacryl-Verbindungen, Vinylpolymere, Polycarbonsäuren, Polyether, Polyimine, Polyamide, Polyurethane

- anorganische Verbindungen, wie zum Beispiel Polykieselsäuren, Tonmineralien wie Montmorillonite, Zeolithe, Kieselsäuren.

Mikrokristalline Cellulose ist ein vorteilhaftes Hydrokolloid im Sinne der erfindungsgemäßen Matrices. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11 % aus Natrium Carboxymethyl Cellulose zusammensetzt. Weitere Handelsprodukte dieser Rohstoffklasse sind Avicel® RC/ CL, Avicel® CE-15, Avicel® 500.

Weitere vorteilhafte Hydrokolloide sind beispielsweise Methylcellulosen, als welche die Methylether der Cellulose bezeichnet werden. Sie zeichnen sich durch die folgende Strukturformel aus

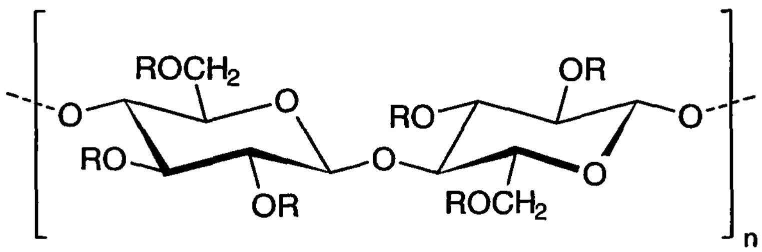

in der R ein Wasserstoff oder eine Methylgruppe darstellen kann.

Insbesondere vorteilhaft im Sinne der erfindungsgemäßen Matrices sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl) methylcellulosen, beispielsweise die unter der Handelsbezeichnung Methocel® E4M bei der Dow Chemical Comp. erhältlichen.

Erfindungsgemäß ferner vorteilhaft ist Natriumcarboxymethylcellulose, das NatriumSalz des Glykolsäureethers der Cellulose, für welches R in der Strukturformel ein Wasserstoff und/oder $CH_2$-COONa darstellen kann. Besonders bevorzugt sind die unter der Handelsbezeichnung Natrosol Plus 330 CS bei Aqualon erhältliche, auch als Cellulose Gum bezeichnete Natriumcarboxymethylcellulose.

Bevorzugt im Sinne der erfindungsgemäßen Matrices ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 $10^6$ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit zum Beispiel 2-Propanol. Xanthan wird anschließend getrocknet und gemahlen.

Vorteilhafter Gelbildner im Sinne der erfindungsgemäßen Matrices ist ferner Carrageen, ein gelbildender und ähnlich wie Agar aufgebauter Extrakt aus nordatlantischen, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens gewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser

leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, μ, ν, ξ π, ω, χ. Auch die Art vorhandener Kationen ($K^+$, $NH_4^+$, $Na^+$, $Mg^{2+}$, $Ca^{2+}$) beeinflusst die Löslichkeit der Carrageene.

Die Verwendung von Chitosan in kosmetischen Zubereitungen ist per se bekannt. Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert beziehungsweise negativ beurteilt werden. Chitosan wird bekanntermaßen beispielsweise in der Haarpflege eingesetzt. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexilcon der Hilfsstoffe für Pharmazie, Kosmetik und angrenzende Gebiete", dritte Auflage 1989, Editio Cantor, Aulendorf, S. 293, Stichwort "Chitosan". Chitosan ist gekennzeichnet durch folgende Strukturformel:

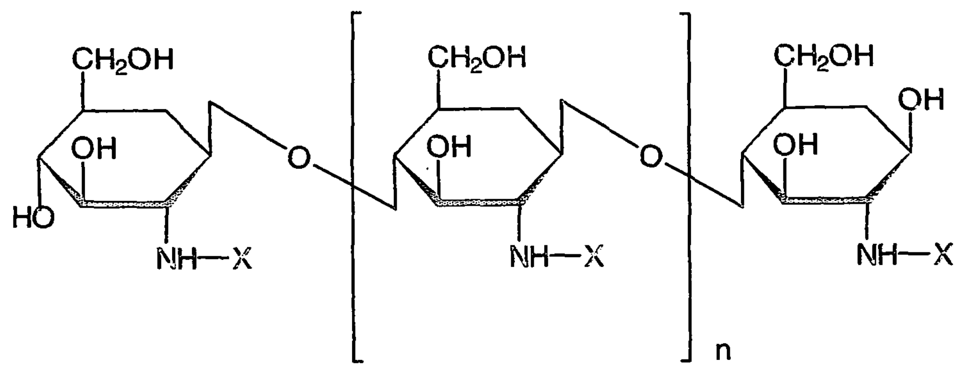

dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel

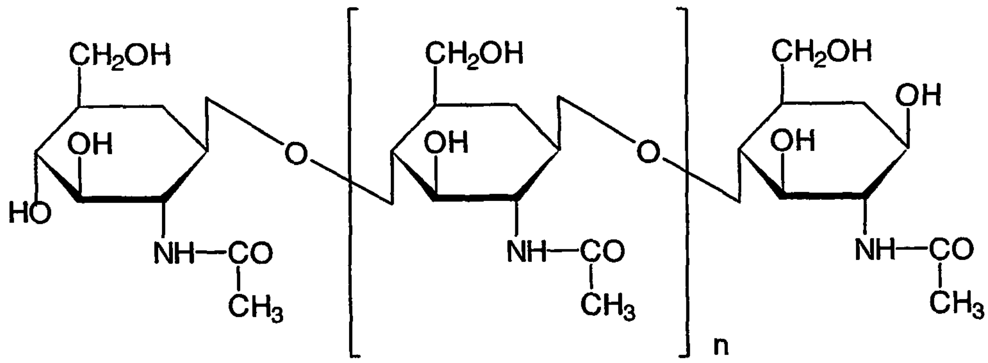

gekennzeichnet ist. Chitin ist wesentlicher Bestandteil des Ektoskeletts [ο χιτων = grch.: der Panzerrock] der Gliederfüßer (zum Beispiel Insekten, Krebse, Spinnen) und wird auch in Stützgeweben anderer Organismen (zum Beispiel Weichtiere, Algen, Pilze) gefunden.

Im Bereich von etwa pH <6 ist Chitosan positiv geladen und dort auch in wässrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP 0 76 657 A1.

Erfindungsgemäß bevorzugt sind Chitosane mit einem Deacetylierungsgrad > 25 %, insbesondere > 55 bis 99 % [bestimmt mittels $^1$H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermeationschromatographie].

**Gelmatrix auf Agar Agar/PAS-Basis**

Die selbstklebende Polymermatrix aus einem in Wasser gelbildenden Polymer umfasst bevorzugt mindestens ein Polyacrylsäurepolymer, Wasser, Meeresalgenextrakt und Alkohol, sowie Silberglas zur desinfizierenden Abgabe von Silberionen an die Haut oder Wunde.
Die Matrix besteht aus einem aus einem in Wasser gelbildenden Polymer, bevorzugt Polyacrylsäuregel, als klebkraftbestimmender Komponente. Als Meeresalgenextrakt wird bevorzugt Agar-Agar eingesetzt. Als Alkohol wird insbesondere ein- oder mehrwertige Alkohole, bevorzugt Glycerin, eingesetzt, die als Konsistenzfaktoren wirken.
Bevorzugt einzusetzender Meeresalgenextrakt ist neben Agar-Agar auch Carrageenan. Carrageenan ist ein hydrophiles Polysaccharid von hohem Molekulargewicht, das aus verschiedenen Rotalgen, vornehmlich Chondrus crispus, durch Heißwasserextraktion, nachfolgendem Ausfrieren und anschließender Reinigung gewonnen wird. Die Struktur von Carrageenan besteht hauptsächlich aus sich wiederholenden Galactose und 3,6 Anhydrogalactoseeinheiten, beide sowohl in sulfatierter wie unsulfatierter Form. Der wichtigste Unterschied zwischen kappa, iota und lambda Carrageenan ist die Anzahl und Position der Estersulfatgruppen an den sich wiederholenden Galactoseeinheiten.
Eine Gelbildung von Carrageenan ist nur in Gegenwart von Kationen möglich. Erfindungsgemäß bevorzugt sind kappa und iota Carrageenan, welche in Gegenwart von Calcium-(kappa und iota), Kalium- und Ammonium-lonen (nur kappa) Gele bilden. Besonders vorteilhaft ist der Einsatz entsprechender Kationenhydroxide, da die zur Herstellung erfindungsgemäßer Gelmatrixsysteme ebenfalls eingesetzte Polyacrylsäure zur Ausbildung stabiler Gele neutralisiert werden muss.

Industriell angeboten wird Carrageenan z.B. von Lehmann & Voss & Co. unter den Bezeichnungen Gelcarin, Viscarin und Seaspen.

Meeresalgenextrakt, wie erfindungsgemäß besonders bevorzugt Agar-Agar, ist ein hydrophiles Kolloid von Polysaccharid-Struldur bestehend aus dem gelierenden Agarose und dem nicht gelierendem Agaropektin, das aus verschiedenen Meeresalgen der Rhodophyceen-Klasse durch Heißwasserextraktion, nachfolgendem Ausfrieren und anschließender Reinigung gewonnen wird. Industriell angeboten wird Agar-Agar z.B. von der Riedel de Haen AG.

Der Extrakt, insbesondere Agar-Agar oder Carrageenan, wird bevorzugt in einer Menge von 0,1 - 15 Gew.%, besonders bevorzugt zwischen 0,5 - 5 Gew.%, eingesetzt. Alle Prozentangaben beziehen sich dabei auf Gewichtsanteile der Polymermatrix sofern nicht Gegenteiliges angegeben ist.
Ein- oder mehrwertige Alkohole wie z.B. Glycerin (1,2,3-Propantriol), sind unter anderem als Lösungsvermittler oder Feuchthaltemittel weit verbreitet eingesetzte Hilfsstoffe der pharmazeutischen Industrie.
Ein- oder mehrwertigen Alkohole, wie z.B. Glycerin, werden erfindungsgemäß bevorzugt in einer Menge von 1 - 85 Gew. %, besonders bevorzugt zwischen 5 - 45 Gew.% eingesetzt.
Der Anteil an in Wasser gelbildendem Polymer wie z.B. Polyacrylsäuregel in der Matrix regelt das Haftvermögen. Im Gegensatz zu Agar-Agar bildet Polyacrylsäure aber sowohl mit Wasser wie auch mit Alkoholen Gele, so dass das durch den Anteil an Polyacrylsäure eingestellte Haftvermögen unabhängig vom jeweiligen Alkoholanteil konstant bleibt.
Der Anteil an Silberglas in der Matrix beträgt bevorzugt 0,001 -10 Gew.%.

Erfindungsgemäß vorteilhafte Polyacrylate sind Acrylat-Alkylacrylat-Copolymere, wie unter der Rubrik Polyacrylsäurematrices beschrieben.

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Das in Wasser gelbildende Polymer, insbesondere Polyacrylsäure und/oder deren Copolymere, werden bevorzugt in einer Menge von 2-55 Gew.%, besonders bevorzugt zwischen 5-30 Gew.% eingesetzt.

Die Herstellung der Polymermatrices erfolgt ohne Verwendung organischer Lösemittel, vorzugsweise bei 40 - 95°C, in handelsüblichen Mischem/Knetem oder kontinuierlich in geeigneten Extrudern.
Als in Wasser gelbildendes Polymer eignet sich u.a. auch Affenbrotbaummehl.
Auf diese Weise lassen sich nur unter Verwendung von Wasser, in Wasser gelbildende Polymer, Meeresalgenextrakt und ein- oder mehrwertigem Alkohol als Ausgangsmaterialien gezielt weiche, geschmeidige, selbstklebende Hydrogelmatrices als Basis zur Herstellung und Anwendung als silberglashaltige Pflaster, TTS, Cataplasmen oder Pads herstellen.
Zur Ausfertigung besonderer anwendungstechnischer Eigenschaften können die Polymermatrices mit entsprechenden Weichmachern, Lösungsvermittlern, Penetrationsenhancern, Neutralisationsmitteln wie z.B. Tromethamol (2-Amino-2-(hydroxymethyl)-1,3-propandiol), Triethanolamin (2,2',2"-Nitrilotriethanol) oder NaOH, Füllstoffen und/oder anderen bekannten Zusätzen versetzt werden, deren Zusatz jedoch nicht zwingend ist.
Die Gelmatrix kann somit mit hydrophilen, bei geeignetem Lösungsvermittler auch hydrophoben, Wirkstoffen zur Wundheilung oder Hautpflege dotiert werden. Bei der Einarbeitung hydrophober Wirkstoffe kann es von Nutzen sein, Cyclodextrine zur Verkapselung einzusetzen.

Cyclodextrine (Cycloamylosen, Cycloglucane) sind in kosmetischen und pharmazeutischen Zubereitungen an sich bekannt.
Die Verbesserung der Löslichkeit schwerlöslicher Substanzen in Gegenwart von Cyclodextrinen in wässrigem Milieu ist

für einzelne Substanzen beschrieben. Vorteilhaft können sowohl die Einschlußverbindungen einer Substanz, auch Gast genannt, mit einer Cyclodextrinspezies, wobei sowohl 1:1 oder 1:2 Komplexe, wie auch Komplexe mit weiteren molaren Verhältnissen (Gast: Cyclodextrin) möglich sind, sowie auch deren physikalische Mischung sein.

Es handelt sich bei den Cyclodextrinen um zyklische Oligosaccharide bestehend aus α-1,4 verknüpften Glucosebausteinen. In der Regel sind sechs bis acht Glucosebausteine (α-, □-, bzw. γ-Cyclodextrin) miteinander verbunden. Cyclodextrine werden bei Einwirkung von *Bacillus macerans* auf Stärke erhalten. Sie besitzen einen hydrophoben Innenraum und eine hydrophile Außenseite. Cyclodextrine und ihre Derivate können aufgrund Ihrer Struktur Inklusionskomplexe bilden. Sie sind zur "molekularen Verkapselung" von Wirkstoffen geeignet (z.B. als schützende Umhüllung empfindlicher Moleküle in kosmetischen und pharmazeutischen Formulierungen).
Diese Anwendungen sind auch in einer Reihe von Patenten beschrieben (z.B.:.WO 98/55148, EP 0 579 435, EP 0 392 608). In diesen Schriften wird jedoch meist nur ein Wirkstoff vom Cyclodextrin (-derivat) komplexiert. Mehrfachkomponenten-Inklusionskomplexe werden zwar in EP 0756 493 beschrieben, doch handelt es sich hier bei näherer Betrachtung um ein Salz und nicht um eine Zweikomponentenmischung von Säure und Base.

Mit "Cyclodextrin und/oder ein Derivat davon" sind im folgenden sowohl Cyclodextrine mit unterschiedlicher Anzahl von Glucosebausteinen im Ringmolekül als auch Derivate dieser Verbindungen gemeint.

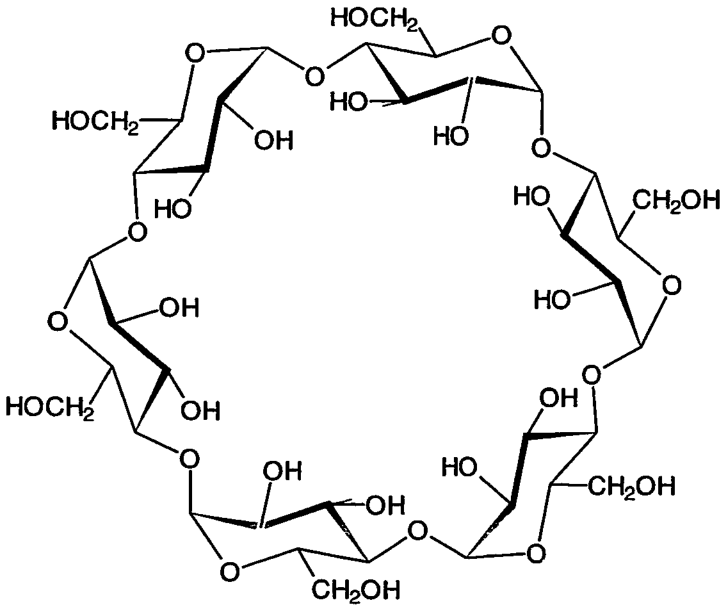

α-Cyclodextrin

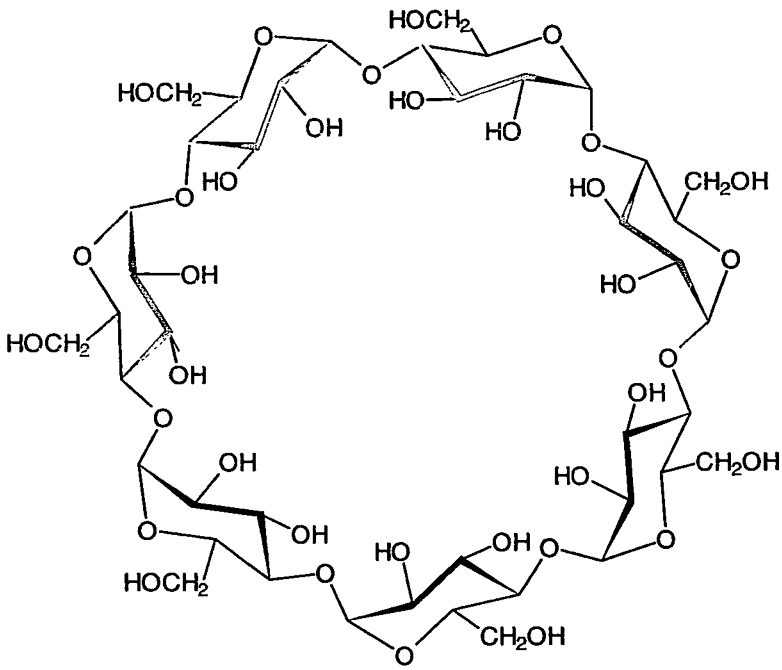

β-Cyclodextrin

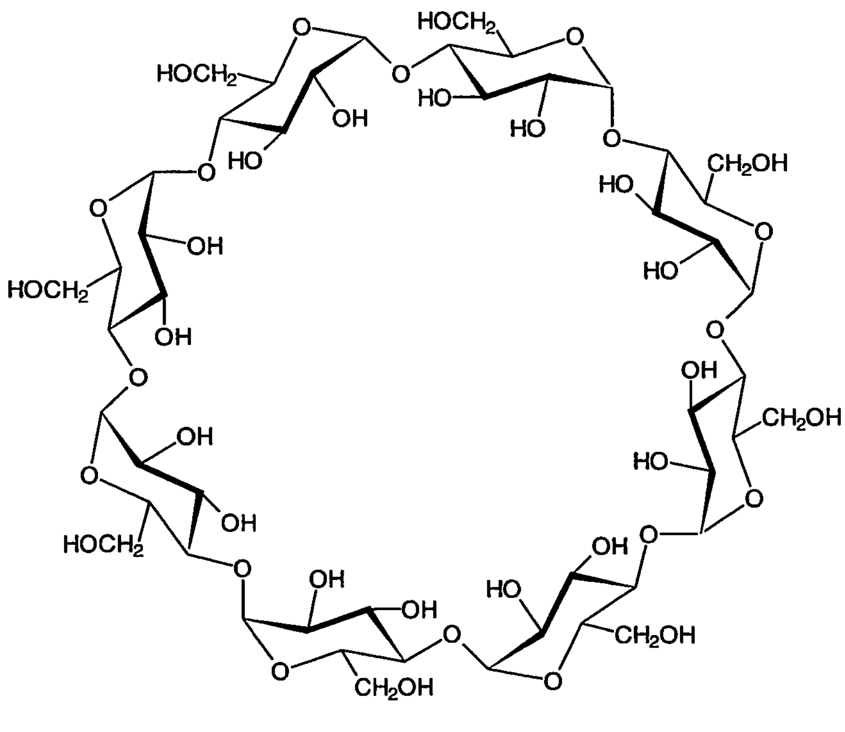

γ-Cyclodextrin

Erfindungsgemäß werden das oder die Cyclodextrine bevorzugt in kosmetischen oder dermatologischen Zusammensetzungen eingesetzt in einer Konzentration von 0,0005 bis 20,0 Gew.%, insbesondere 0,01 bis 10 Gew. % und besonders bevorzugt in einer Konzentration von 0,1 bis 5,0 Gew.%.

Es ist erfindungsgemäß vorteilhaft native, polar- und/oder unpolar- substituierte Cyclodextrine einzusetzen.

Hierzu gehören vorzugsweise aber nicht ausschließlich Methyl-, insbesondere random-Methyl-β-Cyclodextrin, Ethyl- sowie Hydroxypropyl-Cyclodextrine, beispielsweise HP-β-Cyclodextrin oder HP-γ-Cyclodextrin.
Die erfindungsgemäß besonders bevorzugten Cyclodextrinspezies sind γ-Cyclodextrin sowie Hydroxypropyl-β-Cyclodextrin.

Zum weiteren Stand der Technik sind folgende Schriften zu erwähnen:

K. Uekama et al., Chemical Reviews, 1998, 98, 2045-2076, "Cyclodextrin drug carrier systems"
T. Loftsson, Int. J. Dermatology, 1998, 37, 241-246, "Cyclodextrins: new drugdelivery systems in dermatology".
J. Zatz et al. Cosmetics & Toiletries, 1997, 112, Juli, S. 39ff, "Applications of cyclodextrins in skin products.
U. Citemesi, Cosmetics & Toiletries, 1995, 110, März, S. 53 ff, Cyclodextrins in functional dermocosmetics.

Die erfindungsgemäß verwendeten Cyclodextrine bzw. Cyclodextrin-Gast-Inklusionskomplexe bzw. die Cyclodextrin-Substanz Mischungen lassen sich ohne Schwierigkeiten in die Polymermatrix einarbeiten.

In einer erfindungsgemäß besonders bevorzugten Ausführungsform enthält die Polymermatrix bzw. Gelmatrix pharmazeutische Wirkstoffe zur kontrollierten lokalen bzw. systemischen Abgabe an/in die Haut, in Mengen von 0-35 Gew.%, bevorzugt 0-15 Gew.%.
Als Wirkstoffe können beispielsweise ätherische Öle eingesetzt werden. Unter ätherischen Ölen sind aus Pflanzen gewonnene Konzentrate zu verstehen, die als natürliche Rohstoffe hauptsächlich in der Parfüm- und Lebensmittelindustrie eingesetzt werden und die mehr oder weniger aus flüchtigen Verbindungen bestehen. Als Beispiele für diese Verbindungen können 1,8-Cineol, Limonen, Menthol, Borneol und Kampfer genannt werden. Oft wird der Begriff ätherische Öle für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden.
Ätherische Öle bestehen ausschließlich aus flüchtigen Komponenten, deren Siedepunkte in der Regel zwischen 150 und 300 °C liegen. Sie enthalten überwiegend Kohlenwasserstoffe oder monofunktionelle Verbindungen wie Aldehyde, Alkohole, Ester, Ether und Ketone. Stammverbindungen sind Mono- und Sesquiterpene, Phenylpropan-Derivate und längerkettige aliphatische Verbindungen.
Bei manchen ätherischen Öle dominiert ein Inhaltsstoff (zum Beispiel Eugenol in Nelkenöl mit mehr als 85%), andere ätherische Öle stellen hingegen komplex zusammengesetzte Mischungen der einzelnen Bestandteile dar. Oft werden die organoleptische Eigenschaften nicht von den Hauptkomponenten, sondern von Neben- oder Spurenbestandteilen geprägt, wie zum Beispiel von den 1,3,5-Undecatrienen und Pyrazinen im Galbanum-Öl. Bei vielen der kommerziell bedeutenden ätherischen Öle geht die Zahl der identifizierten Komponenten in die Hunderte. Sehr viele Inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

Als bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol genannt werden.

Als weitere ätherische Öle sind Oleum Abietis albae, Oleum Anisi, Oleum Aurantii Floris, Oleum Bergamottae, Oleum Calendulae infusum, Oleum camphoratum, Oleum Caryophylli, Oleum Chamomillae, Oleum Cinnamomi ceylanici, Oleum Citri, Oleum Citronellae, Oleum Cupressi, Oleum Cymbopogonis, Oleum Jecoris, Oleum Lavendulae, Oleum Macidis, Oleum Majoranae, Oleum Melaleucae viridiflorae, Oleum Melissae, Oleum Menthae arvensis, Oleum Menthae piperatae, Oleum Millefolium, Oleum Myrrhae, Oleum Myrte, Oleum Oregani, Oleum Pini sibricum, Oleum Pinisilvestris, Oleum Salviae, Oleum Santali, Oleum Terebinthinae rectificat., Oleum Thymi Oleum Valerianae, Oleum Zingiberis und/oder Teebaumöl zu nennen. Pfefferminzöle sind durch Wasserdampfdestillation aus Blättern und Blütenständen verschiedener Pfefferminze-Sorten gewonnene ätherische Öle, gelegentlich auch solche aus Mentha arvensis.
Citrusöle sind ätherische Öle, die aus den Schalen von Citrusfrüchten (Bergamotte, Grapefruit, Limette, Mandarine, Orange, Zitrone) gewonnen werden, oft auch Agrumenöle genannt.
Citrusöle bestehen zu einem großen Teil aus Monoterpen-Kohlenwasserstoffen, hauptsächlich Limonen (Ausnahme: Bergamottöl, das nur ca. 40% enthält). Beispielsweise kann Menthol zur Oberflächenanästhesierung bei Hautirritationen durch leichte Verbrennungen eingesetzt werden. Die so hergestellten Produkte erzeugen ein angenehmes Kältegefühl und können zur Kühlung von kleineren Verbrennungen, die keiner fachärztlichen Behandlung bedürfen, zum Einsatz kommen.
Menthol hat drei asymmetrische C-Atome und kommt demzufolge in vier diastereomeren Enantiomerenpaaren vor (vgl. die Formelbilder, die anderen vier Enantiomeren sind die entsprechenden Spiegelbilder).

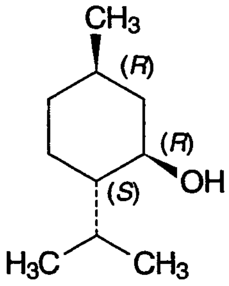

(-)-Menthol
(1)

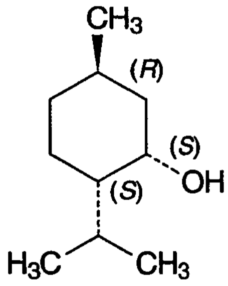

(+)-Neomenthol
(2)

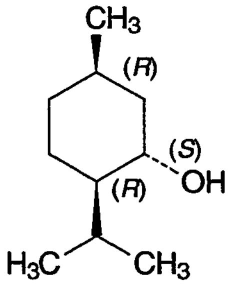

(+)-Isomenthol
(3)

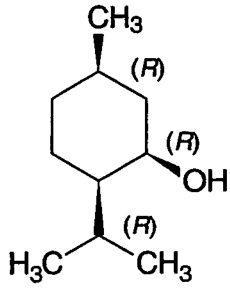

(+)-Neoisomentho
(4)

Die Diastereomeren, die destillativ getrennt werden können, werden als Neoisomenthol, Isomenthol, Neomenthol [(+)-Form: Bestandteil des japanischen Pfefferminzöls] und Menthol bezeichnet. Wichtigstes Isomer ist (-)-Menthol (Levomenthol), glänzende, stark pfefferminzartig riechende Prismen.

Als weitere Wirkstoffe kann zum Beispiel Campher zur Behandlung von rheumatischen Schmeren, Neuralgien und Entzündungen der Matrix zugesetzt werden. Unter Campher versteht man 2-Bornanon, 1,7,7-Trimethylbicyclo[2.2.1] heptan-2-on, siehe untere Abbildung.

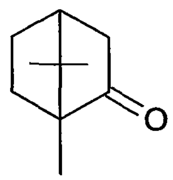

≡

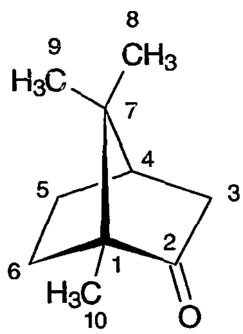

(+)-Campher

Aber auch in Kombination mit pflegenden Substanzen wie Jojobaöl oder Aloe vera ist die erfindungsgemäße Polymermatrix zu verwenden. Solche Kombinationen können je nach Anwendungsdefinition aus einem Arzneimittel ein Kosmetikum machen und somit die Zeit bis zur Vermarktung aufgrund der Verminderung der Zulassungszeiten drastisch verkürzen.

Daneben können für vorteilhafte Ausführungsformen erfindungsgemäßer Hydrogele/Cataplasmen auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika.

Beispielhaft seien genannt:

Capsaicin

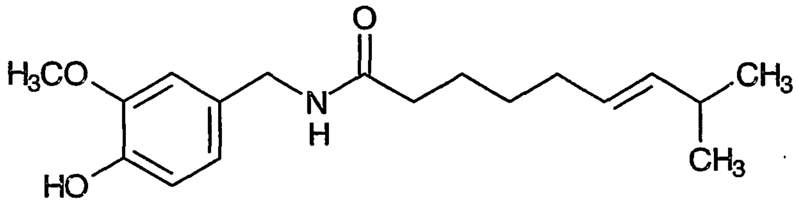

[8-Methyl-trans-6-nonensäure-(4-hydroxy-3- methoxybenzylamid)]

Nonivamid

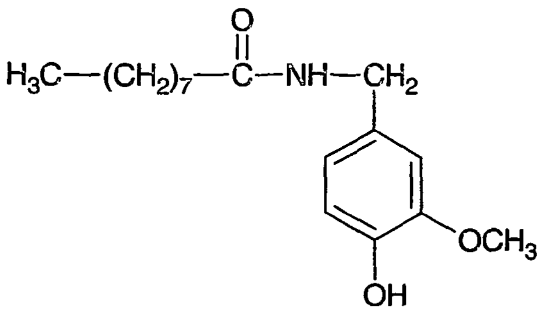

Nicotinsäurebenzylester

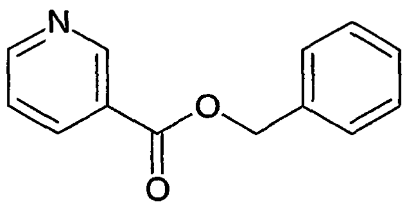

Benzylnicotinat

Auch Flavon und seine Derivate (oft auch kollektiv "Flavon" genannt) sind vorteilhafte Zusatzstoffe im Sinne der vorliegenden Erfindung. Sie sind durch folgende Grundstruktur gekennzeichnet (Substitutionspositionen angegeben):

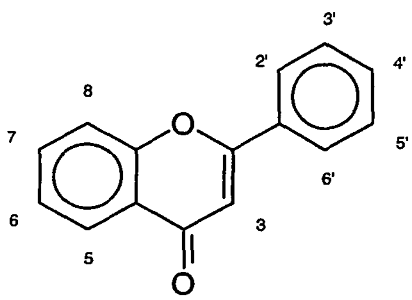

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kampferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel

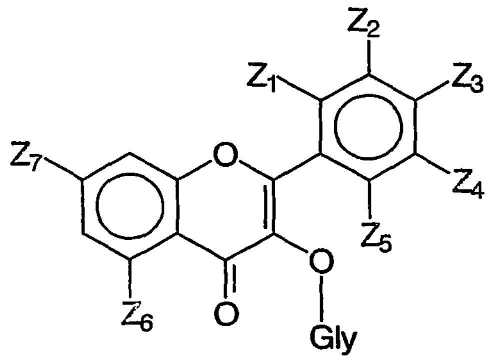

,

wobei $Z_1$ bis $Z_7$ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel

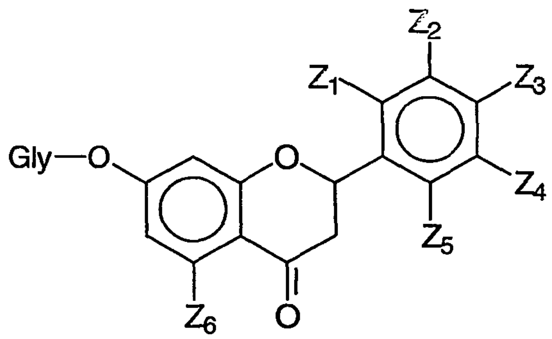

,

wobei $Z_1$ bis $Z_6$ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel

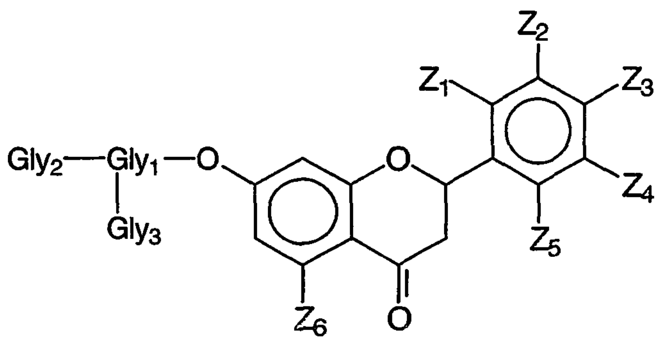

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden $Z_1$ bis $Z_5$ unabhängig voneinander gewählt aus der Gruppe H, OH, Methoxy-, Ethoxy- sowie 2-Hydroxyethoxy-, und die Flavonglycoside haben die Struktur:

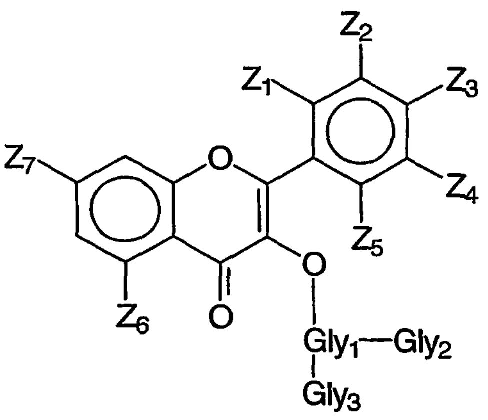

Besonders vorteilhaft werden die erfindungsgemäßen Flavonglycoside aus der Gruppe, welche durch die folgende Struktur wiedergegeben werden:

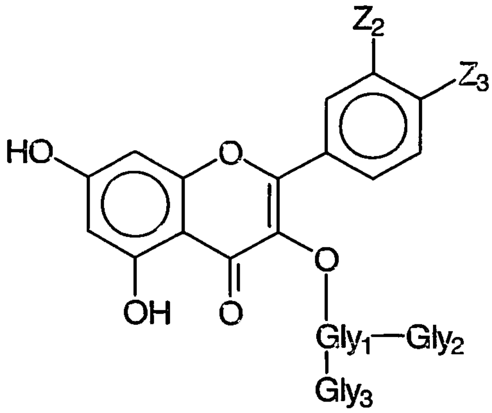

wobei $Gly_1$, $Gly_2$ und $Gly_3$ unabhängig voneinander Monoglycosidreste oder Oligoglycosidreste darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamno-glucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-gtucosid), Flavanomarein (3',4',7,8-Tetrahydroxyflavanon-7-glucosid) und Isoquercetin (3,3',4',5,7-Pentahydroxyflavanon-3-(β-D-Glucopyranosid).

Weitere bevorzugte pharmazeutische Wirkstoffklassen einer erfindungsgemäßen Gelmatrix sind - ohne den Anspruch der Vollständigkeit im Rahmen der vorliegenden Erfindung zu erheben:

Antimykotika, wie z.B. Nafitin, Amorrolfin, Tolnaftat, Ciclopirox
Nichtsteroidale Antirheumatika, wie z.B. Glykolsalicylat, Flufenaminsäure, Ibuprofen, Etofenamat, Ketoprofen, Piroxicam, Indomethacin
Antipuriginosa, wie z.B. Polidocanol, Isoprenalin, Crotamiton
Lokalanästhetika, wie z.B. Lidocain, Benzocain
Antipsoriatika, wie z.B. Ammoniumbitumasulfonat
Keratolytika, wie z.B. Harnstoff

In einer weiteren Ausführungsform kann sich die gegebenenfalls neben Silber auch wirkstoffhaltige Polymermatrix zwischen einer mit ihr fest verankerten Abdeckschicht, auch als Trägerschicht benannt und einer abziehbaren Trennschicht befinden. Die abziehbare Trennschicht dient zur Sicherung der Klebeschicht, zur Verbesserung der Transport- und Lagerstabilität und wird vor dem Applizieren auf die Haut entfernt.
Die Polymermatrix kann auf einer Trägerschicht oder -folie aufgebracht sein, wie sie aus dem Stand der Technik bekannt ist. Die Trägerfolie besteht aus einer luft- und wasserdampfdurchlässigen aber wasserundurchlässigen Polymerschicht mit einer Dicke von ca. 10 bis 100 μm. Die u.U. flexible Trägerfolie besteht vorzugsweise aus Polymeren von Polyurethan, PE, PP, Polyamid, Polyester oder Polyether-ester. Der erfindungsgemäße Wundverband, zumeist in Form eines Pflasters, umfasst eine erfindungsgemäße feucht selbstklebende wirkstoffhaltige Polymermatrix, eine gegebenenfalls wirkstoffundurchlässige Rückschicht und eine ablösbare Schutzschicht, die vor dem Applizieren auf der Haut entfernt wird.

Weitere Ingredienzien, wie Füllstoffe, Stabilisatoren, Enhancer und/oder kosmetische Zusätze können in der Matrix eingebaut werden, um den Verband an die unterschiedlichen Anwendungsgebiete anzupassen und um ein anwendungsfreundlichen Verband bereit zu stellen.

Schließlich kann die Matrix mit einem klebstoffabweisenden Trägermaterial, wie silikonisiertem Papier, eingedeckt oder mit einer Wundauflage oder einer Polsterung versehen werden. Auf seiner feucht selbstklebend ausgerüsteten, später der Haut zugewandten Seite ist der erfindungsgemäße Verband über seine ganze Breite bis zum Gebrauch üblicherweise mit einem Klebstoffabweisenden Trägermaterial abgedeckt. Dieses schützt die Selbstklebeschicht aus der gut hautverträglichen Klebemasse der Matrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Zur Anwendung als Pflaster werden die erfindungsgemäßen Gelmatrices als Schicht auf ein Trennmedium aus Papier, Folie o. ä. gepresst, gewalzt o. ä. und auf der Rückseite mit einem beliebigen Trägermaterial wie z.B. einer Polymerfolie, Textilien o.ä. kaschiert. Erfindungsgemäß besonders bevorzugt werden die Gelmatrices im warmen Zustand mittels Dosierpumpe auf ein Trägermaterial aufgetragen und ganz besonders bevorzugt durch entsprechende Kavitäten in den Press- oder Walzwerken in einer dreidimensionalen Form ausgeführt. Die Form der erzeugten Pflaster wird durch die Form der Kavitäten bestimmt und unterliegt keiner Einschränkung, sie kann z.B. ellipsoid mit flach auslaufenden Rändern oder beispielsweise eckig ausgeführt sein.

Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vorbeziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Besonders vorteilhaft ist, wenn das Trägermaterial sterilisierbar, bevorzugt $\gamma$-(gamma) sterilisierbar, ist.

Erfindungsgemäß ganz besonders bevorzugt sind Trägermaterialien mit einer guten Sauerstoff-, Luft- und Wasserdampfdurchlässigkeit, welche im Siebdruck oder analogen Verfahren punktuell mit der klebenden Polymermatrix versehen sind und an den Seitenrändern die aufgebrachte Gelmatrix nach außen überlappen. Dergestalt ausgefertigte erfindungsgemäße Matrices können an mechanisch stark beanspruchten Körperteilen wie Ellenbogen oder Kniegelenken selbstklebend fixiert werden, wo das eigene Haftvermögen der Hydrogele/Cataplasmen für eine dauerhafte Applikation nicht mehr genügt.

Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe.

Insbesondere die Verwendung der selbstklebenden Polymermatrix, bestehend aus einem Polyacrylsäurepolymer, Agar-Agar, Glycerin und Wasser, in Pflastern, Strips, Wundabdeckungen und/oder Bandagen ist eine äußerst einfache, hautverträgliche Möglichkeit der Wundversorgung bzw. Hautpflege mit desinfizierenden Silberionen aus Silberglas. Die so ausgestatteten Pflaster, Wundabdeckungen oder Bandagen weisen eine individuell einstellbare Konsistenz und Klebkraft auf und sind gegenüber bekannten medizinischen Materialien äußerst wohlfeil. Die Polymermatrix kann alleine oder in Kombination mit geeigneten, beschichteten Trägermaterialien angewendet werden. Es können somit Produkte hergestellt werden, die auch an beweglichen Körperteilen, wie beispielsweise Finger oder Ellenbogen, angewendet werden können.

Des weiteren ist die Verwendung der Polymermatrices, in die wasserlösliche oder hydrophobe Wirkstoffe eingearbeitet sind, als wirkstoffhaltige Pflastersysteme oder TTS zur gezielten Wirkstoffabgabe an die Haut besonders geeignet.

Die Verwendung der selbstklebenden Polymermatrix ist vor allem als wirkstoffhaltige Darreichungsform zur topischen oder buccalen Anwendung oder als Komponente eines, insbesondere monolithischen, TTS vorteilhaft anzusehen.

Beispielsweise kann durch den Einsatz von Menthol auf einem siebdruckbeschichteten Fließstoff ein Pflaster mit der Polymermatrix hergestellt werden, welches durch das Verdampfen, der Abgabe von Menthol und/oder Wasser, zu einem Kühleffekt bei kleinen Verbrennungen führt und durch die abgegebenen Silberionen gleichzeitig das geschädigte Gewebe desinfiziert. Die Verwendung der selbstklebenden silberglashaltigen Polymermatrix enthaltend Menthol als Wirkstoff zur Anwendung bei Hautverbrennungen ist damit bevorzugt. Ebenso erlaubt die Verwendung ätherischer Öle als Wirkstoffe die Anwendung der Polymermatrix bei Erkältungskrankheiten sowie zur Aromatherapie.

Schließlich kann die silberglashaltige Gelmatrix mit einem klebstoffabweisenden Trägermaterial, wie silikonisiertes Papier, eingedeckt oder mit einer Wundauflage oder einer Polsterung versehen werden. Auf seiner selbstklebend ausgerüsteten, später der Haut zugewandten Seite ist das erfindungsgemäße Pflaster über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Gelmatrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Weitere Ausführungsformen können dergestalt sein, dass zwischen der Rückseite der Matrix und dem Abdeckträger sich eine zweite Matrix mit höherer Wirkstofflöslichkeit als Reservoir befindet. Dies könnte statt einer zweiten Matrix und Träger auch eine Tiefziehfolie mit reinem Wirkstoff sein.
Auf der Klebseite der Matrix befindet sich teilweise (z.B. am Rand) eine zweite Matrix mit hoher Klebkraft zur zusätzlichen Fixierung, aber ungenügender Wirkstofflöslichkeit.
Die wirkstofffreie Matrix befindet sich zwischen zwei nicht verankernden Folien und wird zur Fixierung genutzt. Die wirkstofffreie silberglashaltige Matrix könnte auch (mit oder ohne Wundauflage) als Klebschicht für ein einfaches Wund-/ Heftpflaster dienen.

Die Verwendung der silberglashaltigen Polymermatrix als medizinisches Pflastersystem, als Pflaster, Pad, Wundauflage oder Bandage ist besonders in flächiger Ausführungsform mit einer Gesamtfläche von 0,2 bis 1000 $cm^2$ geeignet. Damit werden beispielsweise kleine (0,2 - 2 $cm^2$) Bereiche der Haut abgedeckt oder großflächige Bereiche (bis zu 1000 $cm^2$), beispielsweise zur Kühlung. Bevorzugt ist dabei auch die Verwendung der selbstklebenden silberglashaltigen Polymermatrix in flächiger oder räumlicher Ausführungsform mit einem Polymermatrixgewichtsanteil von 0,1 bis 1000 g, insbesondere von 500 g. Die Form kann dabei rund, oval, eckig oder den Hautpartien angepasst gestaltet sein.

Die Polymer-Matrix, insbesondere eine Polyurethan-Matrix, kann partiell oder vollflächig geschäumt und/oder ungeschäumt, ungefüllt oder mit zusätzlichen Füllstoffen, wie beispielsweise Superabsorbern, Titandioxid, Zinkoxid, Weichmachern, Farbstoffen etc. eingesetzt werden.
Über die Schaumbildung kann ein weicheres Matrixsystem geschaffen werden, das für den Anwender ein positives Anfassgefühl bietet und die Herstellung eines anschmiegsameren Verbandes ermöglicht. Zudem bieten geschäumte Wundauflagen eine Polsterwirkung, was bei druckempfindlicher Anwendung wie z.B. bei Brandverletzungen von Vorteil sein kann.
Vorteilhaft ist aber auch, dass das gesamte Polymermaterial ungeschäumt ist und dennoch hervorragende Anwendungseigenschaften aufweist, da die Schäumungscharakteristik erfindungsgemäß keinen Einfluss auf die Silberfreisetzung hat.

Die Polymermatrix kann gegebenenfalls aus dem Stand der Technik an sich bekannte Zusatzstoffe enthalten, wie zum Beispiel Füllstoffe und Kurzfasem auf anorganischer oder organischer Basis, Metallpigmente, oberflächenaktive Substanzen oder flüssige Streckmittel wie Substanzen mit einem Siedepunkt von über 150°C. Als anorganische Füllstoffe seien beispielsweise Schwerspat, Kreide, Gips, Kieserit, Soda, Titandioxid, Ceroxid, Quarzsand, Kaolin, Russ und Mikrohohlkugeln genannt.

Insbesondere ein Zusatz von Titandioxid mit einem Anteil von 0,01 bis 2 Gew.% bezogen auf die bevorzugte Polyurethanmatrix verbessert den ästhetische Aspekt des silberhaltigen Verbandmaterials in der Art, dass der Anwender beispielsweise kein unästhetisches Blut durch den Verband sehen kann.

An organischen Füllstoffen können zum Beispiel Pulver auf Basis von Polystyrol, Polyvinylchlorid, Hamstoff-Formaldehyd und Polyhydrazodicarbonamid eingesetzt werden. Als Kurzfasern kommen zum Beispiel Glasfasern von 0,1 - 1 mm Länge oder Fasern organischer Herkunft, wie zum Beispiel Polyester- oder Polyamidfasern, in Frage. Metallpulver, wie zum Beispiel Eisen-, Aluminium-, oder Kupferpulver, können ebenfalls bei der Gelbildung mit verwendet werden. Um der Matrix eine gewünschte Färbung zu verleihen, können die bei der Einfärbung von beispielsweise Polyurethanen an sich bekannten Farbstoffe oder Farbpigmente auf organischer oder anorganischer Basis verwendet werden, wie zum Beispiel Eisenoxid- oder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.
Ein Zusatz dieser Farbstoffe ist erfindungsgemäß nicht zwingend erforderlich, da eine Verfärbung der die silberhaltigen Gläser enthaltenden Matrices nicht beobachtet wird. Die Verfärbung mit zusätzlichen Stoffen dient der individuellen Gestaltung des fertigen Polymermaterials um es beispielsweise für Kinder attraktiver zu gestalten sowie der jeweiligen Anpassung an die Umgebungsparameter, wie beispielsweise der Hautfarbe.

Zur Modifizierung der Hafteigenschaften der Polymermatix können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.%, bezogen auf das Gewicht der Polymermasse, zugegeben werden, ohne die vorteilhaften Eigenschaften der Polymermatrix, insbesondere der Polyurethane, zu verwässern.

In die Polymermatrix, die vorteilhaft aus Polyurethan besteht und selbstklebend ausgerüstet ist, wird erfindungsgemäß ein silberhaltiges Glas der beanspruchten Zusammensetzung eingesetzt.

Übliche Glaszusätze, wie beispielsweise andere Metalloxide, die ggf. die Farbe ändern, oder Soda und Pottasche, um den Schmelzpunkt zu erniedrigen, können ggf. zusätzlich enthalten sein.

Das einzusetzende Glas ist vorteilhaft farblos.

Besonders vorteilhaft haben sich Silbergläser der oben angegebenen Zusammensetzung herausgestellt, die eine volumenbezogene Partikelgröße zwischen 0,1 $\mu$m und 10 $\mu$m und einen Restfeuchtegehalt unter 5% besitzen. Erhältlich sind die speziell gefertigten antimikrobiellen Gläser beispielsweise von der Ishizuka Glass Co. Ltd., Japan.

Überraschend wurde gefunden, dass sich die erfindungsgemäßen silberhaltigen Gläser in die Polymermatrix

und insbesondere in eine Polyurethanmatrix durch Zumischen der Gläser zu den Polymerrohstoffen ohne Störung der Reaktion einarbeiten lassen und ihre antimikrobielle oder desinfizierende Wirkung trotz Einbindung in das Polymer entfalten können. Überraschend und nicht vorhersehbar ist weiterhin, dass die Gläser zu keinerlei Eigenschaftsverschlechterung der Polymermatrix beitragen.

Ebenso erstaunlich ist, dass selbst eine Menge von nur 0,005 - 1 Gew.% des Silberglases in das erfindungsgemäße Material, bevorzugt in die Polymermatrix eine antimikrobielle Wirksamkeit zeigt. Des weiteren ist die Wirksamkeit über einen längeren Zeitraum konstant hoch, so dass das Material, beispielsweise als Wundauflage, über einen längeren Zeitraum auf der Haut getragen werden kann ohne Einbussen hinsichtlich der Wirksamkeit hinzunehmen. Insbesondere die hautfreundliche Polyurethanmatrix wirkt dabei synergistisch, so dass auch durch die Polymermatrix keinerlei Nachteile durch eine lange Tragedauer festzustellen sind.

Der herausragendste, nicht vorhersehbare Vorteil ist jedoch, dass die erfindungsgemäße Kombination eines Polymermaterials, das als Wundauflage geeignet ist, mit dem antimikrobiellen Glas zu einer dauerhaften Verfärbungsstabilität der Wundauflage führt.

Das erfindungsgemäße Polymermaterial weist dabei auch eine Verfärbungsstabilität gegenüber Strahlung, Wärme oder sonstigen Einflüssen auf.

Das erfindungsgemäße silberhaltige Polymermaterial weist, je nach Menge an Silberglas, eine Silberfreisetzung von bis zu 50 mg Ag/kg Polymer auf. Bevorzugt ist eine Freisetzungsrate an Silberionen von 5 bis 30 mg/kg, wobei die Freisetzungsrate über die Menge an Silberglas aber auch durch weitere geeignete Zusätze in die Polymermatrix gesteuert werden kann. Die antimikrobielle Wirksamkeit der erfindungsgemäßen Wundauflage ist auch mit geringeren Freisetzungsraten gemäß JIS (Japanischer Industriestandard) 2801:2000 an

- Escherichia coli IFO 3972
- Staphylococus aureus IFO 12732

nachgewiesen worden.

Die Freisetzung an antimikrobiell wirksamen Silberionen aus den erfindungsgemäßen Polymermaterialien wird über einen Zeitraum von 2 bis 240 h, insbesondere von 10 bis 96 h, beobachtet, so dass eine langfristige Behandlung der Wunde mit einer Wundauflage gewährleistet ist. Dieser Vorteil vermeidet den ansonsten häufig erforderlichen Wechsel der Wundauflage.

Die Polymer-Matrix kann vorteilhafterweise transparent eingestellt werden. Als transparent, wasserdampfdurchlässig und klebend erfüllt insbesondere eine Polyurethanmatrix damit ästhetische und anwendungsfreundliche Aspekte. Dies stellt einen signifikanten vorteilhaften Unterschied zu den Polyacylat und Silikongel basierenden Pflastersystemen dar. Die Transparenz erhöht zudem die Akzeptanz beim Anwender, da das Pflaster üblicherweise über einen längeren Zeitraum auf der Haut getragen werden kann.

Zur Speicherung von Flüssigkeit kann vorzugsweise ein Superabsorber-Polymer als Pulver in die Polymermatrix eingearbeitet werden. Hierdurch wird gewährleistet, dass im Bereich der Haut freigesetzte Flüssigkeit gebunden wird, wodurch einer Mazeration und einem vorzeitigen Ablösen des Verbandes entgegen gewirkt wird. Zusätzlich ist durch die erhöhte Aufnahme von Wundsekret und der damit verbundenen verstärkte Absorption von pathogenen Keimen ein erheblicher Produktvorteil bei offenen Wunden gegeben.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE 37 13 601 A1 offenbart werden und auch Superabsorber der neuen Generation mit nur noch geringen Anteilen an austrockenbarem Wasser und hohem Quellvermögen unter Druck. Bevorzugte Produkte sind schwach vernetzte Polymerisate auf der Basis Acrylsäure/Natriumacrylat. Solche Natrium-polyacrylate sind als Favor 22-SK (Stockhausen & Co. KG., Deutschland) erhältlich. Weitere Absorber, zum Beispiel Carboxymethylcellulose und Karaya, sind ebenfalls geeignet.

Es ist daher von Vorteil in das Polymer Superabsorber oder superabsorbieres Polymer in einer Menge von 0,01 bis 40 Gew.%, insbesondere von 0,5 bis 30 Gew.%, insbesondere 20 Gew.%, bezogen auf die Gesamtmasse der Polymer-Matrix, einzuarbeiten.

Eine weitere bevorzugte Ausführungsform sieht vor, dass bei der Herstellung der Polymermatrix und Einbinden des Silberglases auch elementares Aluminium, Zink und/oder Magnesium und/oder deren basische Verbindungen, wie Zinkhydroxid oder Magnesiumchlorid, wasserfrei zugemischt wird.

Dies ermöglicht einerseits hohe Mengen an Silberglaszusatz oder den zusätzlichen Zusatz an antimikrobiellen Silberverbindungen, wie beispielsweise Silberzeolithe, wie er aus dem Stand der Technik bekannt ist. Durch den Zusatz an Aluminium, Zink oder Magnesium bzw. deren basischen Verbindungen wird die zu vermeidende Schwarzfärbung der üblichen Silberverbindungen zusätzlich vermieden.

Durch den Zutritt von Feuchtigkeit über die Wunde oder die Umgebung erfolgt eine Umwandlung des Silbers zu Silberchlorid oder Silberoxid, das wie erwähnt zu unästhetischen Aussehen und Wirksamkeitsverlust bekannter silberhaltiger Verbände führen kann. Der Zusatz an Aluminium, Zink oder Magnesium ermöglicht entsprechend den elektrochemischen Potentialen der Reaktonssysteme

$$2\ AgCl + Zn,\ 2/3\ Al,\ Mag \rightarrow 2\ Ag + ZnCl_2,\ MgCl_2,\ 2/3\ AlCl_3$$

eine Rückbildung des antimikrobiell wirksamen Silbers.

Erfindungsgemäß können die Stoffe Al, Zn, Mg und/oder deren basischen Verbindungen in einer Menge von 0,01 bis 5 Gew.%, bezogen auf die Gesamtmasse des Materials, eingesetzt werden.

Es hat sich gezeigt, dass somit zusätzlich zu den verfärbungsstabilen Silbergläsern auch nichtverfärbungstabile Silberverbindungen in das Polymermaterial eingebaut werden können ohne dass die gewünschte Wirksamkeit und vor allem ästhetische Einbussen hinzunehmen wären.

Die Dicke der Wundauflage kann zwischen etwa 100 bis 2000 μm, vorzugsweise 400 bis 1500 μm, insbesondere zwischen 600 bis 1200 μm liegen.

Ist die erfindungsgemäße Wundauflage selbstklebend ausgerüstet, kann auf zusätzliche Befestigungsmittelverzichtet werden. Die Wundauflage wird direkt als Verbandsmaterial auf die abzudeckende Wunde gelegt und haftet aufgrund der selbstklebenden Eigenschaften auf der die Wunde umgebenden Haut.

Bei größeren Wunden, wenn eine zusätzliche Verklebung erwünscht ist oder wenn die Polymermatrix nicht selbstklebend ausgerüstet ist, kann durch Zusatz einer Randschichtverklebung die Wundauflage auf der Haut verklebt werden. Das erfindungsgemäße Verbandsmaterial ist dann entsprechend bekannten Wundverbände aufgebaut. Sie bestehen im allgemeinen aus einem Trägermaterial, das auf einer Seite mit einer selbstklebenden Schicht versehen ist. Auf diese selbstklebende Beschichtung ist dann die erfindungsgemäße Wundauflage aufgebracht. Um eine einfache Handhabung zu gewährleisten, wird die selbstklebende Beschichtung darüber hinaus mit einer schützenden Schicht eingedeckt, zum Beispiel einem Siegelpapier.

Eine geeignete Klebemasse für die Randschichtverklebung über das zusätzliche Trägermaterial ist in der Schrift DE 27 43 979 C3 dargelegt, weiterhin sind für die Klebebeschichtung bevorzugt handelsübliche druckempfindliche Klebmassen auf Acrylat- oder Kautschukbasis einsetzbar.

Besonders bevorzugt werden thermoplastische Heißschmelzklebemassen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich. Gegebenenfalls kann eine Nachvernetzung durch UV- oder Elektronenstrahlen-Bestrahlung angebracht sein.

**Einarbeitung des Silberglases**

Das Silberglas kann homogen in der gesamten Polymermatrix verteilt sein. Dies wird erreicht, indem es entweder in einer der Ausgangskomponenten der Polymerisation homogen verteilt wird, oder in der fertigen Polymermischung.

Dies kann sowohl in Lösung erfolgen, als auch - aufgrund der guten Hitzebeständigkeit des Silberglases - in der Schmelze (Extruder).

Das Silberglas kann auch durch Schichtaufbau lokal begrenzt in der Matrix verteilt sein, z.B. an der Oberfläche. Dazu kann z.B. eine Polymerlösung, in der das Silberglas enthalten ist, auf die Oberfläche der Polymermatrix aufgebracht werden, beispielsweise gesprüht, extrudiert, gegossen.

Das mit Silberglas versetzte Polymer kann gleichmäßig flächig ausgestrichen werden. Aus dem Ausstrich kann jede beliebig geformte Wundauflage ausgestanzt werden.

Das gleichmäßig flächig ausgestrichene Polymer kann auch Form gebend dahingehend verändert werden, dass die Dicke beliebig variiert wird. Z.B. kann die Mitte der Wundauflage in der ursprünglichen Ausstreichdicke belassen werden, während die Ränder abgeflacht werden.

Das Polymer kann auch von vorneherein nicht flächig ausgestrichen, sondern in beliebige Formen gegossen werden

Die Abmessungen richten sich nach dem beabsichtigten Einsatzgebiet (kleinere Verletzungen z.B. am Finger, größere Verletzungen - z.B. Schürfwunden) und sind frei wählbar.

Ein erfindungsgemäß ausgestattetes Verbandsmaterial, mit oder ohne zusätzlicher Randverklebung, wird wie üblich auf die Wunde aufgelegt.

Die Wundauflage kommt in Kontakt mit dem Wundexudat und nimmt dieses Exudat auf, wodurch ein Aufquellen der Polymermatrix beobachtet wird. Insbesondere bei der Verwendung von Polyurethanmatrices, insbesondere versetzt mit Superabsorbermaterialien, wird eine vorteilhafte Aufnahme des Wundexudates in der Polymermatrix beobachtet. Es können nachfolgend zwei für die Wundheilung wesentliche Schritte identifiziert werden. Einerseits werden nun Silberionen aus dem fein verteilten Glas im Kontakt mit dem Exudat freigesetzt und andererseits können Keime aus der

Wunde in die Polymermatrix aufgenommen werden. Ein erfindungsgemäßer silberglashaltiger Verband wird nach Applikation auf eine exsudierende Wunde durch den Kontakt von Flüssigkeit mit den Silberpartikeln die in der Wundflüssigkeit befindenden Keime abtöten, beziehungsweise eine Kolonisation und unter Umständen eine Infektion der Wunde mit Mikroorganismen unterbinden. Beide Schritte führen allein oder gemeinsam synergistisch zur Verminderung des Keimwachstums und/oder zum Absterben der Keime. Die erfindungsgemäße antimikrobielle Wundauflage hat somit sowohl bakteriostatische als auch bakterizide Eigenschaften, die eine zielgerechte Anwendung antimikrobieller Verbandsmaterialien ermöglicht.

Mit dem Entfernen des silberhaltigen Verbandes ist die antibakterielle Wirkung aufgehoben. Ein Nachwaschen der Wunde zum Entfernen von zuvor temporär applizierten Antiseptika und Antibiotika entfällt.

Die beschriebene Erfindung basiert somit auch auf der beschriebenen antimikrobiellen Wirkung von silberhaltigen Partikeln in Kombination mit einer stark saugenden Wundauflagen, die zusammen einen synergetischen Effekt erreichen. Ferner kann eine Wundauflage, wie etwa die erfindungsgemäße Polyurethan-Wundauflage, über selbstklebende Eigenschaften verfügen, die eine Fixierung auf der intakten Haut am Wundrand des Patienten ermöglichen. Sie betrifft eine neuartige Wundauflage, die zur Behandlung infizierter Wunden oder zum vorbeugenden Schutz vor Wundinfektionen eingesetzt werden können. Dabei bildet der Verband eine Barriere für Mikroorganismen, die das Eindringen von außen verhindert, indem diese beim Kontakt mit der antimikrobiellen Wundauflage abgetötet werden.

Die einzigartige Kombination des hydroaktiven Polyurethan-Polymermaterial mit einer silberhaltigen Glasverbindung ermöglicht eine vorteilhafte Produktamutung für den Konsumenten sowie eine hohe Produktstabilität. Insbesondere wird die unästhetische und vom Endverbraucher nicht akzeptierte Dunkelfärbung, die insbesondere durch Feuchtigkeit, Licht oder $\gamma$-Strahlen ausgelöst wird, wie sie bekannte silberhaltige Materialien aufweisen, signifikant verbessert bzw. sogar ganz vermieden.

Erfindungswesentlich ist dabei, dass sich das antimikrobielle Silberglas ohne Probleme in die Polyurethanmatrix einarbeiten lässt und somit überhaupt erst die Bereitstellung einer antimikrobiellen Wundauflage ermöglicht. Überraschenderweise sind dabei sowohl die Silberglasanteile in dem Polymer, deren Verteilung im Polymer als auch die Anteile an weiteren Zusätze in weiten Bereichen wählbar ohne Einbussen hinsichtlich der geschilderten Vorteile aufzuzeigen.

Weiterhin können zusätzlich hautpflegende oder wundheilende Wirkstoffe in die Polymermatrix eingearbeitet werden, die bei der Applikation auf der Haut die Regeneration der Haut unterstützen. Als Wirkstoffe können Vitamine, wie Vitamin E oder Vitamin C, ätherische Öle, Flavon und seine Derivate, beziehungsweise Antiphlogistika und/oder Analgetika zugesetzt werden.

Das erfindungsgemäße silberhaltige Polymermaterial kann somit als selbstklebende oder mit einer zusätzlichen Randverklebung ausgerüstete Wundauflage in der Wundbehandlung verwendet werden. Des weiteren ist neben der Verwendung in der Wundheilung auch die Verwendung in der Hautpflege, eine Anwendung als Hautschutz sowie die Verwendung als Vorbeugung gegen Hautschädigungen gegeben.

Erfindungsgemäße Polymermaterialien und Wundverbände werden nachfolgend in bevorzugter Ausführung anhand mehrerer Beispiele beschrieben, ohne damit die Erfindung in irgendeiner Weise beschränken zu wollen. Die Anteilsangaben beziehen sich auf die Gesamtmasse an Polymermaterial sofern nichts anderes angegeben ist.

**Kosmetika**

Die vorliegende Erfindung betrifft auch Silberglas enthaltende Kosmetika, insbesondere auf Emulsionen basierende kosmetische, pharmazeutische oder dermatologische Zubereitungen. Die Zubereitungen dienen aufgrund der antimikrobiellen bzw. desinfizierenden Wirkung des Silberglases u.a. zur Prophylaxe und Behandlung von entzündlichen Hautzuständen und/oder zum Hautschutz.

Bei den Silberglas enthaltenden Zubereitungen handelt es sich in der Regel um Emulsionen oder wäßrige Hydrogele, die neben üblichen Feuchthaltesubstanzen auch spezielle Wirkstoffe enthalten können, wie beispielsweise

- ■ entzündungslindernde und kühlende Stoffe,
- ■ lokal anaestesierende Stoffe und/oder
- ■ andere topisch anzuwendende kosmetische, pharmazeutische und/oder dermatologische Wirkstoffe.

Eingesetzt werden z. B. aus Pflanzen gewonnene entzündungslindernde bzw. - hemmende Wirkstoffe wie Azulen und Bisabolol (Kamille), Glycyrrhizin (Süßholzwurzel), Hamamelin (Hamamelis) oder Gesamtextrakte, z. B. aus Aloe vera oder Kamille. Diese zeigen bei leichteren Formen und lokal begrenzten Erythemreaktionen gute Erfolge. Gleiches gilt für Cremes mit einem hohen Gehalt an ätherischen Ölen oder Panthenol.

Aftersun-Präparate sind z. B. dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird beispielsweise durch hohe Mengen an Ethanol erzielt, welches beim Verteilen der Formulierung auf der Haut spontan verdunstet. Auch Hydrogele, O/W-Emulsionen (Lotionen) oder wäßrige Schüttelmixturen haben durch die Verdunstungskälte der wäßrigen Phase einen ausgeprägten Kühleffekt führt. Zur Prophylaxe von entzündlichen Prozessen kann solcherlei Zubereitungen Silberglas zur Desinfektion des geschädigten Gewebes zugesetzt werden.

Die erfindungsgemäßen Formulierungen sind in jeglicher Hinsicht überaus befriedigende Präparate, die sich durch eine hervorragende Wirkung auszeichnen. Bei Anwendung der erfindungsgemäß verwendeten kosmetischen, dermatologischen oder pharmazeutischen Zubereitungen mit einem wirksamen Gehalt an Silberglas ist eine wirksame Behandlung, aber auch eine Prophylaxe von entzündlichen Hautzuständen - auch dem atopischen Ekzem - und/oder zum Hautschutz bei empfindlich determinierter trockener Haut möglich.

Die Erfindung ist selbstverständlich nicht auf topischen Applikationsformen beschränkt, welche nach dem Sonnenbad angewendet werden, sondern umfaßt naturgemäß alle kosmetischen, pharmazeutischen und dermatologischen Anwendungen, bei welchen eine entzündungslindernde Wirkung gewünscht oder von Vorteil sein könnte.

Es ist erfindungsgemäß vorteilhaft, wenn die kosmetischen, pharmazeutischen oder dermatologischen Zubereitungen 0,001 bis 10 Gew.%, insbesondere 0,05 bis 5 Gew.%, ganz besonders 0,1 bis 2 Gew.% an Silberglas enthalten, jeweils bezogen auf das Gesamtgewicht der Zubereitung.

Es ist bevorzugt im Sinne der vorliegenden Erfindung, wenn die erfindungsgemäßen kosmetischen oder dermatologischen zubereitungen einen oder mehrere Alkohole enthalten, insbesondere, wenn die Formulierungen in Form eines Aftersun-Präparats vorliegen und sich durch eine besondere Kühlwirkung auszeichnen sollen.

Es ist weiterhin bevorzugt im Sinne der vorliegenden Erfindung, wenn die erfindungsgemäßen kosmetischen, pharmazeutischen und dermatologischen Zubereitungen mit einem Gehalt von 0,1-30 Gew.%, besonders bevorzugt von 2-10 Gew.%, Glycerin enthalten, um eine gute Befeuchtung der Haut zu gewährleisten.

Die kosmetischen, pharmazeutischen oder dermatologischen Formulierungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Emulsionen können vorzugsweise auch eine Mikroemulsion, eine Pickering-Emulsion oder eine sprühbare Emulsion sein. In diesem Falle könnten pflasterartige Applikationsformen mit ebendiesen Emulsionen getränkt werden. Derartige Emulsionen sind in der Patentanmeldung DE 101 21 092 näher beschrieben, auf die hiermit in vollem Umfang Bezug genommen wird.

Vorzugsweise enthalten die erfindungsgemäßen Formulierungen bzw. Applikationsformen ferner weitere entzündungshemmende Substanzen, wie z. B. Allantoin, α-Bisabolol, Panthotensäure, Panthenol, Gelée Royal, Kamillenextrakte, Azulen oder Aloe-vera-Extrakt sowie unverseifbare Anteile des Avocado- oder Sojaöls und weitere Substanzen, die die gereizte Haut beruhigen. Weitere vorteilhafte Wirkstoffe sind Gerbstoffe, welche adstringierende, entzündungshemmende und/oder sekretionshemmende Wirkung haben.

In einer bevorzugten Ausführungsform der erfindungsgemäßen silberglashaltigen Formulierungen werden diese insbesondere als Aftersun-Hautpflegeprodukte Verwendung finden.

Ein besonders bevorzugtes Anwendungsgebiet der erfindungsgemäßen Zubereitungen liegt in der pflegenden und dekorativen Kosmetik. D.h. es ist möglich auch feste, halbfeste oder stiftförmige kosmetische Zubereitungen für dekorative Zwecke, so genannte Make-up Formulierungen, oder Pflegezubereitungen, wie beispielsweise Lippenpflegestifte, zur Verfügung zu stellen, die ebenfalls eine hervorragende Verteilbarkeit aufweisen und darüber hinaus antimikrobiell bzw. desinfizierend und pflegend wirken.

Bei den stiftförmigen dekorativen Zubereitungen werden hauptsächlich zwei Formulierungsarten unterschieden. Stifte enthalten vornehmlich einen Ölkörper oder neuere Formulierungen sind wasserfrei und benötigen dazu besondere Verdickersysteme, z. B. auf Basis von Mischungen von Stearylalkohol und hydriertem Ricinusöl sowie auf Basis natürlicher oder synthetischer Wachse.

Wasserfreie feste oder halbfeste Formulierungen sind dadurch gekennzeichnet, dass ein oder mehrere feste, in Teilchenform vorliegende Mittel in einem Träger suspendiert sind. Der Träger besteht mindestens aus ein oder mehreren leicht flüchtigen Ölen, ein oder mehreren nicht flüchtigen Emollients und ein oder mehreren Verdickern.

Insbesondere aber legen die Eigenschaften der silberglashaltigen Zubereitungen eine Verwendung in kosmetischen, pharmazeutischen und dermatologischen Produkten dar, die eine Linderung bei gereizten Hautzuständen bzw. die Unterstützung der Wiederherstellung der Hauthomöostase mit einer gleichzeitigen Pflege der Haut verbinden.
Insbesondere ist es für Make-up Produkte vorteilhaft im Sinne der vorliegenden Erfindung, den Zubereitungen gemäß der Erfindung zusätzlich Farbstoffe und/oder -pigmente einzuverleiben.

Bei erfindungsgemäßen silberglashaltigen Applikationsformen können kosmetische oder pharmazeutische Hilfsstoffe enthalten sein, wie sie üblicherweise in solchen Zubereitungen verwendet werden, z. B. Konservierungsmittel, Farbstoffe, Pigmente, die eine färbende Wirkung haben, Lösungsvermittler, Penetrationsbeschleuniger, hydrophile Füllstoffe, Verdickungsmittel, Harze, anfeuchtende und/oder feuchhaltende Substanzen, Fette, Öle, Wachse oder andere übliche Bestandteile einer kosmetischen oder pharmazeutischen Formulierung wie Alkohole, Polyole, Polymere, Schaumstabilisatoren, Elektrolyte, organische Lösungsmittel und/oder Silikonderivate sowie Moisturizer.

Als Verdickungsmittel können beispielsweise vorteilhaft anorganische Gelbildner aus der Gruppe der modifizierten oder unmodifizierten, natürlich vorkommenden oder synthetischen Schichtsilikate gewählt werden.

Es ist zwar durchaus günstig, reine Komponenten einzusetzen, es können jedoch auch in vorteilhafter Weise

Gemische verschiedener modifizierter und/oder unmodifizierter Schichtsilicate den erfindungsgemäßen silberglashaltigen Zubereitungen einverleibt werden.

Zur Anwendung werden die erfindungsgemäßen kosmetischen und dermatologischen Formulierungen in der üblichen Weise, d. h. beispielsweise direkt - nach der Entnahme aus einer Flasche, Tube, einem Tiegel oder einem anderen Behältnis - oder mit Hilfe eines (getränkten) Tuches auf die Haut und/oder die Haare in ausreichender Menge aufgebracht.

Besonderer Vorteil der erfindungsgemäßen Zubereitungen ist, dass die Silbergläser, insbesondere auch durch Ihre vorteilhaften Feinverteilung, zu keinerlei Einbußen in der Anwendung im Vergleich zu gängiger Kosmetika führen. Der erfindungsgemäße Vorteil liegt in der Lagerstabilität und damit Wirksamkeit über einen längeren Zeitraum und insbesondere in der Verfärbungsstabilität durch äußere Einflüsse wie Wärme oder Sonnenlicht. Die erfindungsgemäßen Zubereitungen weisen damit gegenüber anderen Silberhaltigen Kosmetika keine Schwarz- oder Dunkelfärbung auf.

**Tücher, Pads, Hautauflage**

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in der Abwesenheit von (fließendem) Wasser.
Dabei besteht der eigentliche Gebrauchsgegenstand aus zwei Komponenten:

a) einem trockenen Tuch, welches aus Materialien wie Papier und/oder unterschiedlichsten Mischungen aus Natur- oder Kunstfasern aufgebaut ist und
b) einer niederviskosen Tränkungslösung.

Gegenstand der vorliegenden Erfindung sind daher auch kosmetische, pharmazeutische und dermatologische Tücher, welche mit kosmetischen, pharmazeutischen oder dermatologischen Tränkungslösungen befeuchtet sind, die einen Gehalt an Silberglas aufweisen.

Erfindungsgemäß bevorzugte "trockene" Tücher bestehen aus Vlies, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Es hat sich als vorteilhaft herausgestellt für das Tuch, wenn dieses ein Gewicht von 35 bis 120 g/m$^2$, vorzugsweise von 40 bis 60 g/m$^2$, hat (gemessen bei 20 °C $\pm$ 2 °C und bei einer Feuchtigkeit der Raumluft von 65 % $\pm$ 5 % für 24. Stunden).

Die Dicke des Vlieses beträgt vorzugsweise 0,4 mm bis 2 mm, insbesondere 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Fernen weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Es ist vorteilhaft im Sinne der vorliegenden Erfindung, wenn das Gewichtsverhältnis des ungetränkten Tuchs zu der Tränkungslösung aus dem Bereich von 2 : 1 bis 1 : 6 gewählt wird.

Die im Rahmen der Beschreibung der vorliegenden Erfindung genannten erfindungsgemäßen kosmetischen, pharmazeutischen und dermatologischen Formulierungen bzw. Zubereitungen stellen vorteilhafte Tränklösungen für kosmetische und dermatologische Tücher im Sinne der vorliegenden Erfindung dar.

Es ist vorteilhaft, wenn die erfindungsgemäßen Tränklösungen dünnflüssig, insbesondere sprühbar sind und

z. B. eine Viskosität von weniger als 2000 mPa·s, insbesondere weniger als 1.500 mPa·s haben (Meßgerät: Haake Viskotester VT-02 bei 25°C). Die Tränkungslösungen können dabei auch den erfindungsgemäßen kosmetischen Zubereitungen, die das Silberglas enthalten, entsprechen.

**Reinigungszubereitungen**

Erfindungsgemäße Reinigungszubereitungen sind beispielsweise Schaum- und Duschbäder, feste und flüssige Seifen oder sogenannte "Syndets" (synthetische Detergentien), Shampoos, Handwaschpasten, Intimwaschmittel, spezielle Reinigungsmittel für Kleinkinder, Duschgele, Cleansing-Zubereitungen, Make-up-Entferner oder Rasierprodukte. Die Zubereitungen können fest (Seife), niedrigviskos oder gelartig sein, leicht oder stark schäumen und/oder als antibakterielle rinse-off Formulierungen eingesetzt werden. Die Reinigungsprodukte sind sehr mild zur Haut und vorteilhaft ästhetisch transparent. Als Mikroemulsionen können Sie auch als Tränkungsmedium für Tücher, Gewebe dienen, die nass oder trocken vom Verbraucher angewendet werden, wie zuvor beschrieben.

Häufige Erscheinungsformen kosmetischer oder dermatologischer Zubereitungen und Reinigungsprodukten sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 $\mu$m bis ca. 50 $\mu$m. Solche "Makroemulsionen" sind, ohne weitere färbende Zusätze, milchigweißgefärbt und opak. Feinere "Makroemulsionen", deren Tröpfchendurchmesser im Bereich von ca. $10^{-1}$ $\mu$m bis ca. 1 $\mu$m liegen, sind, wiederum ohne färbende Zusätze, bläulichweiß gefärbt und undurchsichtig.

Mizellaren und molekularen Lösungen mit Partikeldurchmessern kleiner als ca. $10^{-2}$ $\mu$m, ist vorbehalten, klar und transparent zu erscheinen.

Der Tröpfchendurchmesser von transparenten bzw. transluzenten Mikroemulsionen dagegen liegt im Bereich von etwa $10^{-2}$ $\mu$m bis etwa $10^{-1}$ $\mu$m. Solche Mikroemulsionen sind meist niedrigviskos. Die Viskosität vieler Mikroemulsionen vom O/W-Typ ist vergleichbar mit der des Wassers.

Vorteil von Mikroemulsionen ist, dass in der dispersen Phase Wirkstoffe feiner dispers vorliegen können als in der dispersen Phase von "Makroemulsionen". Ein weiterer Vorteil ist, dass sie aufgrund ihrer niedrigen Viskosität versprühbar sind.

Die erfindungsgemäßen kosmetischen und dermatologischen Zubereitungen und insbesondere Reinigungszubereitungen können kosmetische Hilfsstoffe enthalten, wie sie üblicherweise in solchen Zubereitungen verwendet werden, z.B. Konservierungsmittel, Bakterizide, Parfüme, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die Herstellung der erfindungsgemäßen kosmetischen und/oder dermatologischen Reinigungszubereitungen erfolgt in der dem Fachmann bekannten üblichen Weise, zumeist dergestalt, dass die erfindungsgemäß verwendeten Substanzen oder Vorlösung dieser Substanzen bei gleichmäßigem Rühren und ggf. unter Erwärmen gelöst bzw. dispergiert werden. Das erfindungsgemäße Silberglas kann dabei vorteilhaft in Mengen von 0,005 bis 10 Gew.%, bezogen auf die Gesamtmasse der Zubereitung, einfach zugesetzt werden.

Durch anschließenden Kontakt mit Wasser werden die Silberionen freigesetzt und entfalten ihre desinfizierende bzw. antimikrobielle Wirkung.

Die nachfolgenden Beispiele erläutern die erfindungsgemäßen Materialien.

Alle Mengenangaben, Anteile und Prozentanteile sind, soweit nicht anders angegeben, auf das Gewicht und die Gesamtmenge bzw. auf das Gesamtgewicht der silberglashaltigen Zubereitungen bezogen.

<u>Beispiel 1</u>

Es wurde ein silberglashaltiges Polymermaterial mit folgender Zusammensetzung hergestellt:

| | |
|---|---|
| Polyetherpolyol (Levagel) : | 16,50 g |
| Vernetzer (Desmodur) : | 1,70 g |
| Vitamin E : | 0,10 g |
| Favor T (Superabsorber) : | 2,05 g |
| Ionpure : | 0,10 g |

(fortgesetzt)

| | |
|---|---|
| Katalysator Coscat : | 0,04 g |
| | 20,50 g |

wobei das silberhaltige Glas Ionopure der Fa. Ishizuka folgende Zusammensetzung ausweist, bezogen auf die Gesamtmasse Glas:

| | Gew.% |
|---|---|
| $P_2O_5$ | 73.35 |
| MgO | 18.33 |
| $Al_2O_3$ | 6.32 |
| $Ag_2O$ | 2.00 |

Beispiel 2

Mit dem in Beispiel 1 hergestellten Polymermaterial wurde die Freisetzung von $Ag^+$-Ionen in 0,9%ige NaCl-Lösung untersucht.

Ein flächig ausgestrichenes Muster A (1 g Probe pro 100 ml 0,9%-ige NaCl-Lösung) der o.g. Zusammensetzung mit einem Flächengewicht von ca. 800 $g/m^2$ setzte dabei folgende in Tabelle 1 dargestellte Mengen Silberionen frei. Vergleichbare Muster aus dem Stand der Technik mit Silberzeolithen (B) bzw. Silberzirkoniumphosphaten (C), an denen in Untersuchungen auch aus dem Stand der Technik antimikrobielle Wirksamkeit nachgewiesen wurde, setzten folgende Mengen Silberionen frei:

Tabelle 1

Freisetzung von Silber aus Polymermaterial gemäß Beispiel 1 (A) im Vergleich zu Standard Verbandmaterialien (B, C)

| Zeit (h) / Silberfreisetzung (mg/kg) | Verbandsmaterial | | |
|---|---|---|---|
| | A | B | C |
| 24 | 23,8 | 14,4 | 28,5 |
| 72 | 25,4 | 25,0 | 23,4 |
| 168 | 28,3 | 26,5 | 29,6 |

Das Beispiel zeigt, dass eine Freisetzung von Silberionen aus dem erfindungsgemäßen Polymermaterial in der selben Größenordnung beobachtet wird wie aus dem Stand der Technik bekannten antimikrobiellen Verbandsmaterialien mit Silberzeolithen (B) bzw. Silberzirkoniumphosphaten (C).

Beispiel 3

Herstellung von erfindungsgemäßen Wundauflagen mit verschiedenen Gehalt an Silberglas.

Muster D:

Es wurde eine Wundauflage mit folgender Zusammensetzung hergestellt (0,01 Gew.% Silberglas):

| | |
|---|---|
| Polyetherpolyol (Levagel) : | 14,505 g |
| Vernetzer (Desmodur) : | 1,391 g |
| Vitamin E : | 0,057 g |
| Favor T (Superabsorber) : | 4,524 g |
| Ionpure : | 0,002 g |
| Coscat 83 : | 0,041 g |
| | 20,520 g |

Muster E

Es wurde eine Wundauflage mit folgender Zusammensetzung hergestellt (0,05 Gew.% Silberglas):

| | |
|---|---|
| Polyetherpolyol (Levagel) : | 14,41 g |
| Vernetzer (Desmodur) : | 1,38 g |
| Vitamin E : | 0,06 g |
| Favor T (Superabsorber) : | 4,50 g |
| Ionpure : | 0,01 g |
| Coscat : | 0,04 g |
| | 20,39 g |

Muster F

Es wurde eine Wundauflage mit folgender Zusammensetzung hergestellt (0,075 Gew.% Silberglas):

| | |
|---|---|
| Polyetherpolyol (Levagel) : | 14,41 g |
| Vernetzer (Desmodur) : | 1,38 g |
| Vitamin E : | 0,06 g |
| Favor T (Superabsorber) : | 4,51 g |
| Ionpure : | 0,016 g |
| Coscat : | 0,04 g |
| | 74<br>20,41 g |

Muster G

Es wurde eine Wundauflage mit folgender Zusammensetzung hergestellt (0,1 Gew.% Silberglas):

| | |
|---|---|
| Polyetherpolyol (Levagel) : | 79,03 g |
| Vernetzer (Desmodur) : | 7,65 g |
| Vitamin E : | 0,30 g |
| Favor T (Superabsorber) : | 22,76 g |
| Ionpure : | 0,11 g |
| Coscat : | 0,36 g |
| | 110,22g |

Muster H

Es wurde eine Wundauflage mit folgender Zusammensetzung hergestellt (0,25 Gew.% Silberglas):

| | |
|---|---|
| Polyetherpolyol (Levagel) : | 78,68 g |
| Vernetzer (Desmodur) : | 7,57 g |
| Vitamin E : | 0,30 g |
| Favor T (Superabsorber) : | 22,66 g |
| Ionpure : | 0,28 g |
| Coscat : | 0,36 g |
| | 109,86g |

Muster I

Es wurde eine Wundauflage mit folgender Zusammensetzung hergestellt (0,52 Gew.% Silberglas):

| | |
|---|---|
| Polyetherpolyol (Levagel) : | 78,95 g |
| Vernetzer (Desmodur) : | 7,58 g |
| Vitamin E : | 0,31 g |
| Favor T (Superabsorber) : | 22,74 g |
| Ionpure : | 0,57 g |
| Coscat : | 0,36 g |
| | 110,51 g |

Muster J

Es wurde eine Wundauflage mit folgender Zusammensetzung hergestellt (1,02 Gew.% Silberglas):

| | |
|---|---|
| Polyetherpolyol (Levagel) : | 79,16 g |
| Vernetzer (Desmodur) : | 7,55 g |
| Vitamin E : | 0,30 g |
| Favor T (Superabsorber) : | 22,91 g |
| Ionpure : | 1,14 g |
| Coscat : | 0,36 g |
| | 111,42g |

Beispiel 4

Die hergestellten Muster D - J (ca. 800g/$m^2$) des Beispiels 3 wurden bezüglich ihrer Freisetzung von Silberionen nach 24h gemäß folgender Vorschrift untersucht.
Eine 30$cm^2$ große Probe wurde bei 32°C in 100ml isotonischer Kochsalzlösung eingelegt. Nach 24h wurden die Proben entnommen und die Lösung über einen 0,45μm Membranfilter filtriert und die Konzentration des Silbers in der Lösung über Graphitrohr-AAS bestimmt.
Die folgende Tabelle und Abbildung 1 zeigen zusammenfassend die Ergebnisse der Silberfreisetzung.

| Probenbezeichnung | Gew.% Silberglas | Konzentration Ag |
|---|---|---|
| | | [mg Ag/kg Polymermaterial] |
| D | 0,01 | 1,5 |
| E | 0,05 | 5,2 |
| F | 0,075 | 9,4 |
| G | 0,1 | 13,0 |
| H | 0,25 | 20,0 |
| I | 0,52 | 22,0 |
| J | 1,02 | 20,0 |

Beispiel 5

Die hergestellten Muster D bis J wurden bzgl. ihrer antimikrobiellen Wirksamkeit gemäß JIS 2801:2000 an

- Escherichia coli IFO 3972
- Staphylococus aureus IFO 12732

untersucht.
Die Aktivität der Proben wird gemäß der folgenden Gleichung (1) berechnet:

$$\text{Antimicrobial activity} = \log_{10} \frac{\text{Number of living bacteria at beginning}}{\text{Number of living bacteria after } 24\,h} \quad (1)$$

Von einer antimikrobiellen Aktivität kann demnach ausgegangen werden, wenn die Aktivität >2 ist, d.h. die Anzahl der untersuchten Bakterien um den Faktor 100 reduziert wird.
Es zeigte sich gemäß Tabelle 2, dass alle untersuchten Muster eine ausreichende antimikrobielle Aktivität besitzen.

Tabelle 2: Antimikrobielle Aktivität

| Muster | $Ag_{Freisetzung}$ nach 24h | Antimikrobielle Aktivität gemäß (1) | |
|---|---|---|---|
| | [mg/mg] | Escherichia coli | Staphylococus aureus |
| D | | | |
| G | 13 | > 3,6 | > 3,3 |
| I | 22 | > 3,6 | > 3,3 |
| I steril | 20 | > 3,6 | >3,3 |

Beispiel 6

Zum Vergleich der Verfärbungsstabilität der erfindungsgemäßen Wundauflagen wurden die Muster D bis J auf ihre Änderung der Farbe durch Zusatz des Silberglases untersucht. Abbildung 2 zeigt die Wundauflagen gemäß des Beispiels 3 als s/w Kopie. Man erkennt keine Änderung gegenüber der undotierten Referenz bis hin zu Muster H. Erst bei einer Konzentration oberhalb von 0,25 Gew.% Silberglas (Muster H) lässt sich eine leichte, nur schwer mit bloßem Auge erkennbare Farbänderung erkennen.

Beispiel 7

Um die Stabilität des Silberglases beim Sterilisieren zu prüfen, wurde das Muster J mit 26 kGy $\gamma$-sterilisiert. Wie in der Abbildung 3 zu erkennen ist, führt die $\gamma$-Sterilisation zu keiner Farbveränderung. Eine $\gamma$ - Sterilisation des fertigen Pflasters führte zu keinerlei Einbussen hinsichtlich der antimikrobiellen Wirksamkeit gemäß JIS Z 2801:2000 und, was außerordentlich erstaunlich ist, zu keiner Verfärbung des Verbandsmateriales.

Beispiel 8

Um die Alterungsstabilität der erfindungsgemäßen Wundverbände zu prüfen, wurden Muster G sechs Monate bei 50°C einer beschleunigten Alterung unterzogen und hinsichtlich der Farbstabilität begutachtet. Auch hierbei wurden keinerlei Farbveränderungen festgestellt, was selbst durch die abgebildeten s/w Kopien der Abbildung 4 noch deutlich erkennbar wird.

Beispiel 9

Angaben zur Polymermatrix

Folgende Eigenschaften (zusätzlich zur Dicke) zur Charakterisierung schlage ich vor:

| | | |
|---|---|---|
| Flüssigkeitsaufnahme: | 0.5-10 | g/g |
| | bevorzugt 1.0 - 6 | g/g |
| | bes. bevorzugt 1.5 - 3.5 | g/g |

Methode:

Eine runde Probe mit einem Durchmesser von 22 mm wird ausgestanzt und eine Stunde bei 23 ± 2 °C und 50 ± 5 % r.F. vorkonditioniert. Die Proben werden gewogen und für 3 Stunden vollständig in physiologische 23 ± 0,5

°C warme Natriumchloridlösung getaucht. Die Proben werden erneut gewogen und die Flüssigkeitsaufnahme aus der Wägedifferenz berechnet.

| | |
|---|---|
| Wasserdampfdurchlässigkeit: | 100-5000 g/($m^2$*24h) |
| | bevorzugt 250 - 2500 g/($m^2$*24h) |
| | bes. bevorzugt 300 - 1500 g/($m^2$*24h) |

Methode:

Die Prüfung erfolgt nach ASTM E 96 (water method), mit folgenden Abweichungen:

Die Öffnung des Prüfgefäßes beträgt 804 $mm^2$
Das Material wird 24 Std. vorkonditioniert bei 23 ± 2 °C und 50 ± 5 % r.F.
Der Abstand zwischen Wasserspiegel im Prüfgefäß und der Probe beträgt 35 ± 5 mm
Das Rückwiegen der mit Proben bestückten Prüfgefäße erfolgt nach 24 Std., in denen diese im Klimaschrank bei 37 ± 1,5 °C und 30 ± 3 % r.F. gelagert werden.

Beispiel 10 - Polyisobutylenmatrices

| | |
|---|---|
| Vistanex LM MH: | 48,33 Gew.% |
| Vistanex MM L80: | 28,00 Gew.% |
| Eastoflex PLS E1003D | 10,00 Gew.% |
| Cetiol V | 13,17 Gew.% |
| Silberglas | 0,50 Gew.% |

Beispiel 11 - Polyacrylsäurematrices

| | |
|---|---|
| Polyacrylsäure | 22,5 Gew.% |
| Polyvinylpyrrolidon, PVP 25 | 3,5 Gew.% |
| Propandiol | 37,49 Gew.% |
| Polyethylenglycol | 20,0 Gew.% |
| Kieselsäure | 11,5 Gew.% |
| Dexpanthenol | 5,0 Gew.% |
| Silberglas | 0,01 Gew.% |

Beispiel 12 - Silikonmatrices

| | |
|---|---|
| Carbopol | 32,0 Gew.% |
| Q7-9600 A | 23,4 Gew.% |
| Q7-9600 B | 13,9 Gew.% |
| Silikon-PSA | 29,7 Gew.% |
| Silberglas | 1,0 Gew.% |

Beispiel 13 - Kautschukmatrices

| | |
|---|---|
| Silberglas | 1,000 % |

(fortgesetzt)

| | |
|---|---|
| Colan 46 | 2,099 % |
| Keromet MD 100 | 0,262 % |
| Füllstoff IR | 13,120% |
| Crepe | 11,546 % |
| Ameripol 1011 | 21,591% |
| Natsyn 2200 | 8,397 % |
| Harz 115 | 11,021% |
| Harz 95 | 9,971 % |
| Harz SE 10 | 8,922 % |
| Gelböl | 7,347 % |
| Wollwachs DAB | 4,724 % |

Beispiel 14 - SBC-Hotmelt Matrices

| | |
|---|---|
| Kraton D-1113, Kraton: | 43,87 Gew.% |
| Escorez 5380, Exxon: | 24,54 Gew.% |
| Sylvares TR 7115, Arizona | 21,90 Gew.% |
| Whitemor WOM 14, Castrol | 3,84 Gew.% |
| Cetiol V, Henkel | 4,35 Gew.% |
| Irganox 1010, Ciba-Geigy | 0,78 Gew.% |
| Silberglas | 0,72 Gew.% |

Beispiel 15 - Polymerfolie auf PVA/PAS-Basis

| | |
|---|---|
| Mowiol 18/88 | 73,40 Gew.% |
| Carbopol 980 | 16,00 Gew.% |
| Dexpanthenol | 5,00 Gew.% |
| Lutrol E 400 | 5,00 Gew.% |
| Q 10 | 0,50 Gew.% |
| Silberglas | 0,10 Gew.% |

Beispiel 16-20: PIT - Sprays

| | **16** | **17** | **18** | **19** | **20** |
|---|---|---|---|---|---|
| Glycerinmonostearat SE | 0,50 | | 3,00 | 2,00 | 4,00 |
| Ceteareth-12 | | 5,00 | | 1,00 | 1,50 |
| Ceteareth-20 | | | | 2,00 | |
| Ceteareth-30 | 5,00 | | 1,00 | | |
| Stearyl Alkohol | | | 3,00 | | 0,50 |
| Cetyl Alkohol | 2,50 | 1,00 | | 1,50 | |
| Ethylhexyl Methoxycinnamat | | | | 5,00 | 8,00 |
| Aniso Triazin | | 1,50 | | 2,00 | 2,50 |

(fortgesetzt)

| | 16 | 17 | 18 | 19 | 20 |
|---|---|---|---|---|---|
| Butyl Methoxydibenzoylmethan | | | 2,00 | | |
| Dioctyl Butamidotriazon | 1,00 | 2,00 | | 2,00 | |
| Ethylhexyl Triazon | 4,00 | | 3,00 | 4,00 | |
| 4-Methylbenzyliden Camphor | | 4,00 | | | 2,00 |
| Octocrylen | | 4,00 | | | 2,50 |
| Bisimidazylat | | | 0,50 | | 1,50 |
| Phenylbenzmidazol Sulfonsäure | 0,50 | | | 3,00 | |
| $C_{12-15}$ Alkyl Benzoat | | 2,50 | | | 5,00 |
| Titandioxid | 0,50 | 1,00 | | 3,00 | 2,00 |
| Zinkoxid | 2,00 | | 3,00 | 0,50 | 1,00 |
| Dicaprylyl Ether | | | 3,50 | | |
| Butylen Glycol Dicaprylat/Dicaprat | 5,00 | | | 6,00 | |
| Dicaprylyl Carbonat | | | 6,00 | | 2,00 |
| Dimethicon | | 0,50 | 1,00 | | |
| Phenyltrimethicon | 2,00 | | | 0,50 | 0,50 |
| Shea Butter | | 2,00 | | | 0,50 |
| PVP Hexadecen Copolymer | 0,50 | | | 0,50 | 1,00 |
| Glycerin | 3,00 | 7,50 | 5,00 | 7,50 | 2,50 |
| Vitamin E Acetat | 0,50 | | 0,25 | | 1,00 |
| Silberglas | 0,30 | 0,10 | 0,60 | 0,20 | 0,30 |
| Alpha Glucosylrutin | 0,10 | | 0,20 | | |
| DMDM Hydantoin | 0,60 | | 0,40 | 0,20 | |
| Koncyl-L ® | | 0,20 | | | 0,15 |
| Methylparaben | | 0,50 | 0,25 | 0,15 | |
| Phenoxyethanol | 0,50 | 0,40 | | 1,00 | 0,60 |
| Ethanol | 3,00 | 2,00 | 1,50 | | 1,00 |
| Parfum | q.s. | q.s. | q.s. | q.s. | q.s. |
| Wasser | ad.100 | ad.100 | ad.100 | ad.100 | ad.100 |

Beispiel 21 - 25; O/W Cremes

| | 21 | 22 | 23 | 24 | 25 |
|---|---|---|---|---|---|
| Glyceryl Stearat Citrat | | | 2,00 | | 2,00 |
| Glyceryl Stearat SE | | 3,00 | | | |
| Cetearyl Alcohol + PEG-40 Castor Oil + So- | 3,00 | | | | |
| dium Cetearyl Sulfate | | | | | |
| Polyglyceryl-3-Methylglucose Distearat | | | | 3,00 | |

(fortgesetzt)

| | 21 | 22 | 23 | 24 | 25 |
|---|---|---|---|---|---|
| Sorbitan Stearat | | | | | 2,00 |
| Stearinsäure | | 1,00 | | | |
| Stearyl Alkohol | | | 5,00 | | |
| Cetyl Alkohol | | 2,00 | | 3,00 | |
| Cetylstearylalkohol | | | | | 2,00 |
| Caprylic/Capric Triglycerid | 5,00 | 3,00 | 4,00 | 3,00 | 3,00 |
| Octyldodecanol | | | 2,00 | | 2,00 |
| Dicaprylether | | 4,00 | | 2,00 | 1,00 |
| Mineralöl | | 2,00 | | 3,00 | |
| Cyclomethicon | 3,00 | | | | |
| $TiO_2$ | | | 1,00 | | |
| 4-Methylbenzyliden Camphor | | | 1,00 | | |
| Butyl Methoxydibenzolymethan | | | 0,50 | | |
| Silberglas | 0,30 | 0,30 | 0,50 | 0,10 | 1,00 |
| Tocopherol | 0,20 | | | | 0,20 |
| Hydroxipropyl Methylcellulose | 0,30 | | | | |
| Trisodium EDTA | | | 0,10 | 0,1 | |
| Konservierungsmittel | | q.s. | q.s. | q.s. | q.s. |
| Xanthan Gummi | | | | | |
| Carbomer | 0,30 | 0,1 | | 0,1 | 0,1 |
| Natronlauge 45% | q.s. | q.s. | q.s. | q.s. | q.s. |
| Glycerin | 6,00 | 3,00 | 4,00 | 3,00 | 3,00 |
| Ethylhexylglycerin | 0,25 | | | | |
| Butylen Glycol | | 3,00 | | | |
| Alkohol Denat. | 7,0 | | | | |
| Parfum | q.s. | q.s. | q.s. | q.s. | q.s. |
| Aqua | ad 100 | ad 100 | ad 100 | ad 100 | ad 100 |

Beispiel 26-30; O/W Cremes

| | 26 | 27 | 28 | 29 | 30 |
|---|---|---|---|---|---|
| Glyceryl Stearat Citrat | | 2,00 | 2,00 | | |
| Glyceryl Stearat SE | 5,00 | | | | |
| Stearinsäure | | | | 2,50 | 3,50 |
| Stearyl Alkohol | 2,00 | | | | |
| Cetyl Alkohol | | | | 3,00 | 4,50 |
| Cetylstearylalkohol | | 3,00 | 1,00 | | 0,50 |

(fortgesetzt)

| | 26 | 27 | 28 | 29 | 30 |
|---|---|---|---|---|---|
| $C_{12-15}$Alkyl Benzoat | | 2,00 | 3,00 | | |
| Caprylic/Capric Triglycerid | 2,00 | | | | |
| Octyldodecanol | 2,00 | 2,00 | | 4,00 | 6,00 |
| Dicaprylether | | | | | |
| Mineralöl | | 4,00 | 2,00 | | |
| Cyclomethicon | | | | 0,50 | 2,00 |
| Dimethicon | 2,00 | | | | |
| $TiO_2$ | 2,00 | | | | |
| 4-Methylbenzyliden Camphor | 1,00 | | | | 1,00 |
| Butyl Methoxydibenzolymethan | 0,50 | | | | 0,50 |
| Silberglas | 0,10 | 0,30 | 0,20 | 0,10 | 0,20 |
| Tocopherol | | | | | 0,05 |
| Trisodium EDTA | | | 0,20 | | 0,20 |
| Konservierungsmittel | q.s. | q.s. | q.s. | q.s. | q.s. |
| Xanthan Gummi | | | 0,20 | | |
| Carbomer | 0,15 | 0,1 | | 0,05 | 0,05 |
| Natronlauge 45% | q.s. | q.s. | q.s. | q.s. | q.s. |
| Glycerin | 3,00 | | 3,00 | 5,00 | 3,00 |
| Butylen Glycol | | 3,00 | | | |
| Alkohol Denat. | | 3,00 | | 3,00 | |
| Parfum | q.s. | q.s. | q.s. | q.s. | q.s. |
| Aqua | ad 100 | ad 100 | ad 100 | ad 100 | ad 100 |

Beispiel 31 - 37; W/O Emulsionen

| | 31 | 32 | 33 | 34 | 35 | 36 | 37 |
|---|---|---|---|---|---|---|---|
| Cetyldimethicon Copolyol | | 2,50 | | 4,00 | | | |
| Polyglyceryl-2-dipolyhydroxystearat | 5,00 | | | | 4,50 | 4,00 | 5,00 |
| PEG-30-dipolyhydroxystearat | | | 5,00 | | | | |
| Lanolin Alkohol | | | | | | 0,50 | 1,50 |
| Isohexadecan | | | | | | 1,00 | 2,00 |
| Myristyl Myristat | | | | | | 0,50 | 1,50 |
| Cera Microcristallina + Paraffinum Liquidum | | | | | | 1,00 | 2,00 |
| Ethylhexyl Methoxycinnamat | | 8,00 | | 5,00 | 4,00 | | |
| Aniso Triazine | 2,00 | 2,50 | | 2,00 | 2,50 | | |
| Butyl Methoxydibenzoylmethan | | | 2,00 | 1,00 | | 0,50 | 1,50 |

(fortgesetzt)

| | 31 | 32 | 33 | 34 | 35 | 36 | 37 |
|---|---|---|---|---|---|---|---|
| Dioctyl Butamidotriazon | 3,00 | 1,00 | | | 3,00 | | |
| Ethylhexyl Triazon | | | 3,00 | 4,00 | | | |
| 4-Methylbenzylidene | | 2,00 | | 4,00 | 2,00 | 1,00 | 3,00 |
| Camphor | | | | | | | |
| Octocrylen | 7,00 | 2,50 | 4,00 | | 2,50 | | |
| Dioctylbutamidotriazon | 1,00 | | | 2,00 | | | |
| Bisimidazylat | 1,00 | 2,00 | 0,50 | | | | |
| Phenylbenzmidazole Sulfo- 0,50 | | | | 3,00 | 2,00 | | |
| nic Acid | | | | | | | |
| Titandioxid | | 2,00 | 1,50 | | 3,00 | | |
| Zinkoxid | 3,00 | 1,00 | 2,00 | 0,50 | | | |
| Mineralöl | | | 10,0 | | 8,00 | | |
| $C_{12-15}$ Alkyl Benzoat | | | | 9,00 | | | |
| Dicaprylyl Ether | 10,00 | | | | 7,00 | | |
| Butylene Glycol Dicapry- | | | 2,00 | 8,00 | 4,00 | 4,00 | 5,00 |
| lat/Dicaprat | | | | | | | |
| Dicaprylyl Carbonat | 5,00 | | 6,00 | | | | |
| Dimethicone | | 4,00 | 1,00 | 5,00 | | | |
| Cyclomethicone | 2,00 | 25,00 | | | 2,00 | | |
| Shea Butter | | | 3,00 | | | | 0,50 |
| PVP Hexadecen Copolymer | 0,50 | | | 0,50 | 1,00 | | |
| Butylen Glycol | | | | | | | 6,00 |
| Octoxyglycerin | | 0,30 | 1,00 | | 0,50 | | 3,00 |
| Glycerin | 3,00 | 7,50 | | 7,50 | 2,50 | 5,00 | |
| Glycin Soja | | 1,00 | 1,50 | | | | |
| $MgSO_4$ | 1,00 | 0,50 | | 0,50 | | | |
| $MgCl_2$ | | | 1,00 | | 0,70 | | |
| Vitamin E Acetat | 0,50 | | 0,25 | | 1,00 | 0,50 | 1,00 |
| Silberglas | 0,10 | 0,30 | 0,20 | 0,40 | 0,30 | 1,00 | 0,60 |
| Trisodium EDTA | | | | | | 0,20 | 0,20 |
| DMDM Hydantoin | | 0,60 | 0,40 | 0,20 | | | |
| Methylparaben | q.s. | q.s. | q.s. | q.s. | q.s. | q.s. | q.s. |
| Phenoxyethanol | q.s. | q.s. | q.s. | q.s. | q.s. | q.s. | q.s. |
| Ethanol | 3,00 | | 1,50 | | 1,00 | | 3,00 |
| Parfum | q.s. | q.s. | q.s. | q.s. | q.s. | q.s. | q.s. |
| Wasser | ad 100 | ad 100 | ad 100 | ad 100 | ad 100 | ad 100 | ad 100 |

Beispiel 38 - 42; Hydrodispersionen

| | 38 | 39 | 40 | 41 | 42 |
|---|---|---|---|---|---|
| Ceteareth-20 | 1,00 | | | 0,5 | |
| Cetyl Alkohol | | | 1,00 | | |
| Natrium Carbomer | | 0,20 | | 0,30 | |
| Acrylates/C10-30 Alkyl Acrylate Crosspoly- | 0,50 | | 0,40 | 0,10 | 0,10 |
| mer | | | | | |
| Xanthan Gummi | | 0,30 | 0,15 | | 0,50 |
| Ethylhexyl Methoxycinnamat | | | | 5,00 | 8,00 |
| Aniso Triazin | | 1,50 | | 2,00 | 2,50 |
| Butyl Methoxydibenzoylmethan | 1,00 | | 2,00 | | |
| Dioctyl Butamidotriazon | | 2,00 | | 2,00 | 1,00 |
| Ethylhexyl Triazon | 4,00 | | 3,00 | 4,00 | |
| 4-Methylbenzyliden Camphor | 4,00 | 4,00 | | | 2,00 |
| Octocrylen | | 4,00 | 4,00 | | 2,50 |
| Dioctylbutamidotriazon | 1,00 | | | 2,00 | |
| Bisimidazylat | 1,00 | | 0,50 | | 2,00 |
| Phenylbenzmidazol Sulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| $C_{12-15}$ Alkyl Benzoat | 2,00 | 2,50 | | | |
| Dicaprylyl Ether | | 4,00 | | | |
| Butylene Glycol Dicaprylat/Dicaprat | 4,00 | | 2,00 | 6,00 | |
| Dicaprylyl Carbonat | | 2,00 | 6,00 | | |
| Dimethicone | | 0,50 | 1,00 | | |
| Phenyltrimethicone | 2,00 | | | 0,50 | 2,00 |
| Shea Butter | | 2,00 | | | |
| PVP Hexadecene Copolymer | 0,50 | | | 0,50 | 1,00 |
| Octoxyglycerin | | | 1,00 | | 0,50 |
| Glycerin | 3,00 | 7,50 | | 7,50 | 2,50 |
| Glycin Soja | | | 1,50 | | |
| Vitamin E Acetat | 0,50 | | 0,25 | | 1,00 |
| Silberglas | 0,30 | 0,10 | 0,50 | 0,30 | 0,20 |
| DMDM Hydantoin | | 0,60 | 0,40 | 0,20 | |
| Koncyl - L ® | q.s. | q.s. | q.s. | q.s. | q.s. |
| Methylparaben | q.s. | q.s. | q.s. | q.s. | q.s. |
| Phenoxyethanol | q.s. | q.s. | q.s. | q.s. | q.s. |
| Ethanol | 3,00 | 2,00 | 1,50 | | 1,00 |

(fortgesetzt)

| | 38 | 39 | 40 | 41 | 42 |
|---|---|---|---|---|---|
| Parfum | q.s. | q.s. | q.s. | q.s. | q.s. |
| Wasser | ad 100 | ad 100 | ad 100 | ad 100 | ad 100 |

Beispiel 43 Gelcreme

| | Massengehalt (%) |
|---|---|
| Acrylat/C10-30 Alkyl Acrylat Crosspolymer | 0,40 |
| Carbomer | 0,20 |
| Xanthan Gummi | 0,10 |
| Cetearyl Alkohol | 3,00 |
| $C_{12-15}$ Alkyl Benzoate | 4,00 |
| Caprylic/Capric Triglycerid | 3,00 |
| Cyclometicone | 5,00 |
| Dimeticone | 1,00 |
| Silberglas | 0,30 |
| Glycerin | 3,00 |
| Natriumhydroxid | q.s. |
| Konservierung | q.s. |
| Parfum | q.s. |
| Wasser,demineralisiert | ad 100,0 |
| pH-Wert eingestellt auf 6,0 | |

Beispiel 44 W/O-Creme

| | |
|---|---|
| Lameform TGI | 3,50 |
| Glycerin | 3,00 |
| Dehymuls PGPH | 3,50 |
| Silberglas | 0,10 |
| Konservierungsmittel | q.s. |
| Parfum | q.s. |
| Wasser, demin. | ad 100,0 |
| Magnesiumsulfat | 0,6 |
| Isopropyl Stearate | 2,0 |
| Caprylyl Ether | 8,0 |
| Cetearyl Isononanoate | 6,0 |

Beispiel 45 W/O/W-Creme

| | Massengehalt (%) |
|---|---|
| Glyceryl Stearat | 3,00 |
| PEG-100 Stearat | 0,75 |
| Behenylalkohol | 2,00 |

(fortgesetzt)

| | Massengehalt (%) |
|---|---|
| Caprylic/Capric Triglycerid | 8,0 |
| Octyldodecanol | 5,00 |
| $C_{12-15}$ Alkyl Benzoate | 3,00 |
| Silberglas | 1,00 |
| Magnesium Sulfat ($MgSO_4$) | 0,80 |
| EDTA | 0,10 |
| Konservierung | q.s. |
| Parfum | q.s. |
| Wasser,demineralisiert | ad 100,0 |
| pH-Wert eingestellt auf 6,0 | |

## Patentansprüche

**1.** Material geeignet zum Auftragen oder Applizieren auf die Haut enthaltend antimikrobiell wirksames silberhaltiges Glas der Zusammensetzung

| | |
|---|---|
| $P_2O_5$ | 40 - 60 mol%, |
| $R^1O$ | 35 - 55 mol%, |
| $R^2{}_2O$ | 0-5 mol%, |
| $SiO_2$, $Al_2O_3$ | 5-20 mol%, bezogen auf die Gesamtmenge des silberoxidfreien Glases, und |
| $Ag_2O$. | 0,1 bis 5 Gew.%, bezogen auf die Gesamtmasse des Glases, |

wobei $R^1$gewählt wird aus Ca, Mg, Zn und/oder Cu und $R^2$ gewählt wird aus Na, K und/oder Li und wobei das Material gewählt ist

a.) aus der Gruppe der Polynnermaterialien Polyacrylate, Polyisobutylen, Styrolblockcopolymere (SBC), SIBS-Massen, SEBS-Massen, Silikone. Kautschukmassen, Chitosane, Alginate, Hydrogele, Hydrokolloide, Gelmatrices auf Agar Agar/PAS-Basis, Polymeren auf PVA/PAS-Basis und/oder Polyurethane,
b.) aus der Gruppe der kosmetischen Zubereitungen, Emulsionen, insbesondere in Form von W/O-, O/W-, W/O/W- oder O/W/O-Emulsionen, Mikroemulsion, Pickering-Emulsion oder sprühbare Emulsion sowie wässrige Hydrogele,
c.) aus der Gruppe der Tücher, Pads, Hautauflagen, wobei die Materialien mit einer das Silberglas enthaltenden Tränkungslösung befeuchtet sind, oder
d.) aus der Gruppe der Reinigungszubereitungen, insbesondere Schaum- und Duschbader, feste und flüssige Seifen oder so genannte "Syndets" (synthetische Detergentien), Shampoos, Handwaschpasten, Intimwaschmittel, spezielle Reinigungsmittel für Kleinkinder, Duschgele, Cleansing-Zubereitungen, Make-up-Entferner oder Rasierprodukte.

**2.** Material nach Anspruch 1 mit einer Glaszusammensetzung

| | |
|---|---|
| $P_2O_5$ | 45 - 55 mol%, |
| CaO, MgO | 35-50 mol%, |
| $Na_2O$, $K_2O$ | 0 - 5 mol%, |
| $SiO_2$ | 0 - 5 mol%, |
| $Al_2O_3$ | 5 - 15 mol%, bezogen auf die Gesamtmenge des silberoxidfreien Glases, und |
| $Ag_2O$ | 0,5 - 3 Gew.%, bezogen auf die Gesamtmasse des Glases. |

**3.** Material nach einem der vorstehenden Ansprüche, **dadurch gekennzeichnet, dass** 0,001 bis 40 Gew.%, bevorzugt 0,05 bis 1 Gew.%, des silberhaltigen Glases, bezogen auf die Gesamtmasse des Materials, enthalten sind.

4. Material nach einem der vorstehenden Ansprüche, **dadurch gekennzeichnet, dass** die Silbergläser eine volumenbezogene Partikelgröße zwischen 0,1 μm und 10 μm besitzen.

5. Material nach einem der vorstehenden Ansprüchen, **dadurch gekennzeichnet, dass** die Silbergläser einen Restfeuchtegehalt unter 5% besitzen.

6. Material nach einem der vorstehenden Ansprüche, **dadurch gekennzeichnet, dass** das Material aus der Gruppe der Polymermaterialien a.) gewählt wird.

7. Polymermaterial nach Anspruch 6, **dadurch gekennzeichnet, dass** das Polymermaterial selbstklebend ausgerüstet ist.

8. Polymermaterial nach einem der vorstehenden Ansprüche 6 oder 7, **dadurch gekennzeichnet, dass** das Polymermaterial transparent ist

9. Polymermaterial nach einem der vorstehenden Ansprüche 6 bis 8, **dadurch gekennzeichnet, dass** in der Polymermatrix zusätzlich Superabsorber, bevorzugt zu einem Anteil von 0,01 bis 40 Gew.%, insbesondere 0,5 bis 30 Gew.%, insbesondere 20 Gew.%, bezogen auf die Gesamtmasse der Polymermatrix, enthalten sind.

10. Polymermaterial nach einem der vorstehenden Ansprüche 6 bis 9, **dadurch gekennzeichnet, dass** zusätzlich weitere antimikrobielle Silberverbindungen enthalten sind.

11. Polymermaterial nach einem der vorstehenden Ansprüche 6 bis 10, **dadurch gekennzeichnet, dass** hautpflegende und/oder wundheilende Wirkstoffe zugesetzt sind.

12. Polymermaterial nach einem der vorstehenden Ansprüche 6 bis 11, **dadurch gekennzeichnet, dass** das Polymermaterial als Verbandsmaterial ausgebildet ist und auf der hautzugewandten Seite eines Tragermaterials aufgebracht ist, das auf der hautzugewandten Seite mit einer selbstklebenden Schicht versehen ist.

13. Verwendung der Materialien nach einem der Ansprüche 1 bis 12 zur Herstellung eines Arzneimittels zur antimikrobiellen oder desinfizierenden Haut-, Haar- und/oder Wundpflege.

14. Verwendung der Materialien nach einem der Ansprüche 1 bis 12 als Kosmetikum zur antimikrobiellen oder desinfizierenden Haut-, und/oder Haarpflege.

15. Verwendung des Polymermaterials nach Ansprüche 13 als hydroaktive Wundauflage für die feuchte Wundbehandlung.

16. Verwendung des Polymermaterials nach einem der Ansprüche 13 oder 14 als antimikrobielle, gegenüber Strahlung, Feuchtigkeit und/oder Warme verfärbungsstabile Wundauflage zur Applikation auf der menschlichen Haut.

17. Verwendung der kosmetischen Zubereitung oder Reinigungszubereitung nach Anspruch 14 zur Linderung bei gereizten Hautzuständen, Unterstützung der Wiederherstellung der Hauthomöostase und gleichzeitiger Pflege der Haut.

18. Verwendung der kosmetischen Zubereitung nach Anspruch 14 als After-Sun Produkt.

## Claims

1. Material suitable for placement or application to the skin, comprising antimicrobial, silver-containing glass of composition

| | |
|---|---|
| $P_2O_5$ | 40-60 mol%, |
| $R^1O$ | 35-55 mol%, |
| $R^2{}_2O$ | 0- 5 mol%, |
| $SiO_2$, $Al_2O_3$ | 5-20 mol%, based on the total amount of the silver oxide-tree glass, and |

(continued)

| | |
|---|---|
| $Ag_2O$ | 0.1% to 5% by weight, based on the total mass of the glass, $R^1$ being selected from Ca, Mg, Zn and/or Cu and $R^2$ being selected from Na, K and/or Li and the material being selected. |

a.) from the group of the polymer materials polyacrylates, polyisobutylene, styrene block copolymers (SBC), SIBS compounds, SEBS compounds, silicones, rubber compounds, chitosans, alginates, hydrogels, hydrocolloids, gel matrices based on agar agar/PAS, polymers based on PVA/PAS, and/or polyurethanes,
b.) from the group of cosmetic preparations, emulsions, especially in the form of W/O, O/W, W/O/W or O/W/O emulsions, a microemulsion, Pickering emulsion or sprayable emulsion, and aqueous hydrogels,
c.) from the group of wipes, pads, skin contact materials, the materials being moistened with an impregnating solution comprising the silver glass, or
d.) from the group of cleansing preparations, especially bath foams and shower products, solid and liquid soaps or what are called "syndets" (synthetic detergents), shampoos, handwash pastes, intimate washes, special cleansing products for infants, shower gels, cleansers, makeup removers or shaving products.

**2.** Material according to Claim 1, having a glass composition

| | |
|---|---|
| $P_2O_5$ | 45-55 mol%, |
| CaO, MgO | 35-50 mol%, |
| $Na_2O$, $K_2O$ | 0- 5 mol%, |
| $SiO_2$ | 0- 5 mol%, |
| $Al_2O_3$ | 5-15 mol%, based on the total amount of the silver oxide-free glass, and |
| $Ag_2O$ | 0.5%-3% by weight, based on the total mass of the glass. |

**3.** Material according to either of the above claims, **characterized in that** 0.001% to 40% by weight, preferably 0.05% to 1% by weight, of the silver-containing glass is present, based on the total mass of the material.

**4.** Material according to any one of the above claims, **characterized in that** the silver glasses possess a volume-based particle size of between 0.1 $\mu$m and 10 $\mu$m.

**5.** Material according to any one of the above claims, **characterized in that** the silver glasses possess a residual moisture content below 5%.

**6.** Material according to any one of the above claims, **characterized in that** the material is selected from the group of the polymer materials a.).

**7.** Polymer material according to Claim 6, **characterized in that** the polymer material is self-adhesive.

**8.** Polymer material according to either of Claims 6 and 7 above, **characterized in that** the polymer material is transparent.

**9.** Polymer material according to any one of Claims 6 to 8 above, **characterized in that** there are additionally present in the polymer matrix superabsorbents, preferably in a fraction of 0.01% to 40% by weight, in particular 0.5% to 30% by weight, especially 20% by weight, based on the total mass of the polymer matrix.

**10.** Polymer material according to any one of Claims 6 to 9 above, **characterized in that** further antimicrobial silver compounds are additionally present.

**11.** Polymer material according to any one of Claims 6 to 10 above, **characterized in that** active skincare and/or wound-healing substances are added.

**12.** Polymer material according to any one of Claims 6 to 11 above, **characterized in that** the polymer material is in the form of dressing material and is applied on the skin-facing side of a backing material which on the skin-facing side is provided with a self-adhesive layer.

**13.** Use of the materials according to any one of Claims 1 to 12 for preparing a medicament for antimicrobial or disinfectant skincare, haircare and/or woundcare.

**14.** Use of the materials according to any one of Claims 1 to 12 as cosmetic for antimicrobial or disinfectant skincare and/or haircare.

**15.** Use of the polymer material according to Claim 13 as hydroactive wound contact material for moist wound treatment.

**16.** Use of the polymer material according to either of Claims 13 and 14 as antimicrobial wound contact material, stable to discoloration with respect to radiation, moisture and/or heat, for application to the human skin.

**17.** Use of the cosmetic preparation or cleansing preparation according to Claim 14 for alleviating irritated skin conditions, assisting the reestablishment of dermal homeostasis, and simultaneously caring for the skin.

**18.** Use of the cosmetic preparation according to Claim 14 as an aftersun product.

**Revendications**

**1.** Matériau approprié à l'étalement ou l'application sur la peau, contenant un verre contenant de l'argent à action antimicrobienne, de composition

| | |
|---|---|
| $P_2O_5$ | 40-60 % en moles, |
| $R^1O$ | 35-55 % en moles, |
| $R^2{}_2O$ | 0-5 % en moles, |
| $SiO_2$, $Al_2O_3$ | 5-20 % en moles, par rapport à la quantité totale du verre sans oxyde d'argent, et |
| $Ag_2O$ | 0,1 à 5 % en poids, par rapport à la masse totale du verre, |

$R^1$ étant choisi parmi Ca, Mg, Zn et/ou Cu et $R^2$ étant choisi parmi Na, K et/ou Li et le matériau étant choisi

a) dans le groupe des matériaux polymères polyacrylates, polyisobutylène, copolymères séquencés de styrène (SBC), matières SIBS, matières SEBS, silicones, matières de type caoutchouc, chitosanes, alginates, hydrogels, hydrocolloïdes, matrices de gel à base d'agar-agar-PAS, polymères à base de PVA/PAS et/ou polyuréthannes,
b) dans le groupe des préparations cosmétiques, émulsions, en particulier sous forme d'émulsions E/H, H/E, E/H/E ou H/E/H, microémulsion, émulsion de Pickering ou émulsion pulvérisable ainsi qu'hydrogels aqueux,
c) dans le groupe des lingettes, emplâtres, dispositifs transdermiques, les matériaux étant imprégnés avec une solution d'imprégnation contenant le verre à l'argent, ou
d) dans le groupe des préparations de nettoyage, en particulier des produits pour la douche et moussants, des savons liquides et savons solides ou des dénommés "syndets'" (détergents synthétiques), des shampooings, pâtes lavantes pour les mains, produits pour la toilette intime, produits de nettoyage spéciaux pour enfants en bas âge, gels de douche, préparations lavantes, démaquillants ou produits de rasage.

**2.** Matériau selon la revendication 1, ayant une composition de verre

| | |
|---|---|
| $P_2O_5$ | 45-55 % en moles, |
| CaO, MgO | 35-50 % en moles, |
| $Na_2O$, $K_2O$ | 0-5 % en moles, |
| $SiO_2$ | 0-5 % en moles, |
| $Al_2O_3$ | 5-15 % en moles, par rapport à la quantité totale du verre sans oxyde d'argent, et |
| $Ag_2O$ | 0,5-3 % en poids, par rapport à la masse totale du verre. |

**3.** Matériau selon l'une quelconque des revendications précédentes, **caractérisé en ce que** sont contenus 0,001 à 40 % en poids, de préférence 0,05 à 1 % en poids, du verre contenant de l'argent, par rapport à la masse totale du matériau.

4. Matériau selon l'une quelconque des revendications précédentes, **caractérisé en ce que** les verres à l'argent ont une taille de particule, sur la base du volume, comprise entre 0,1 µm et 10 µm.

5. Matériau selon l'une quelconque des revendications précédentes, **caractérisé en ce que** les verres à l'argent présentent une teneur en humidité résiduelle inférieure à 5 %.

6. Matériau selon l'une quelconque des revendications précédentes, **caractérisé en ce que** le matériau est choisi dans le groupe des matériaux polymères a.).

7. Matériau polymère selon la revendication 6, **caractérisé en ce que** le matériau polymère est traité pour être autoadhésif.

8. Matériau polymère selon l'une quelconque des revendications précédentes 6 ou 7, **caractérisé en ce que** le matériau polymère est transparent.

9. Matériau polymère selon l'une quelconque des revendications précédentes 6 à 8, **caractérisé en ce qu'**en outre des superabsorbants sont contenus dans la matrice polymère, de préférence en une proportion de 0,01 à 40 % en poids, en particulier de 0,5 à 30 % en poids, plus particulièrement de 20 % en poids, par rapport à la masse totale de la matrice polymère.

10. Matériau polymère selon l'une quelconque des revendications précédentes 6 à 9, **caractérisé en ce qu'**en outre d'autres composés antimicrobiens contenant de l'argent sont contenus.

11. Matériau polymère selon l'une quelconque des revendications précédentes 6 à 10, **caractérisé en ce que** des substances actives cicatrisantes et/ou pour le soin de la peau sont ajoutées.

12. Matériau polymère selon l'une quelconque des revendications précédentes 6 à 11, **caractérisé en ce que** le matériau polymère est constitué sous forme de matériau de pansement et est appliqué du côté tourné vers la peau d'un matériau de support qui est muni d'une couche autoadhésive sur la face tournée vers la peau.

13. Utilisation des matériaux selon l'une quelconque des revendications 1 à 12, pour la fabrication d'un médicament destiné au soin antimicrobien ou désinfectant de la peau, des cheveux et/ou de plaies.

14. Utilisation des matériaux selon l'une quelconque des revendications 1 à 12, en tant que cosmétique pour le soin antimicrobien ou désinfectant de la peau et/ou des cheveux.

15. Utilisation du matériau polymère selon la revendication 13 en tant que pansement de plaies hydroactif pour le traitement humide de plaies.

16. Utilisation du matériau polymère selon l'une quelconque des revendications 13 ou 14 en tant que pansement de plaies antimicrobien, à coloration stable vis-à-vis d'irradiation, de l'humidité et/ou de la chaleur, pour application sur la peau humaine.

17. Utilisation de la préparation cosmétique ou de la préparation de nettoyage selon la revendication 14, pour le soulagement d'états cutanés irrités, la facilitation du rétablissement de l'homéostasie de la peau et le soin simultané de la peau.

18. Utilisation de la préparation cosmétique selon la revendication 14, en tant que produit après-soleil.

Abbildung 1

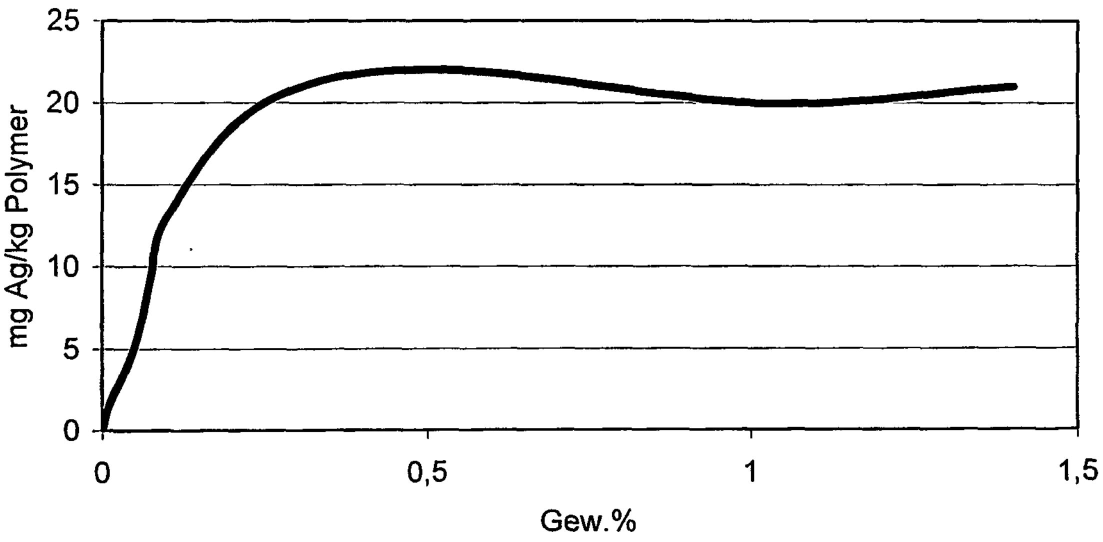

Abbildung 2

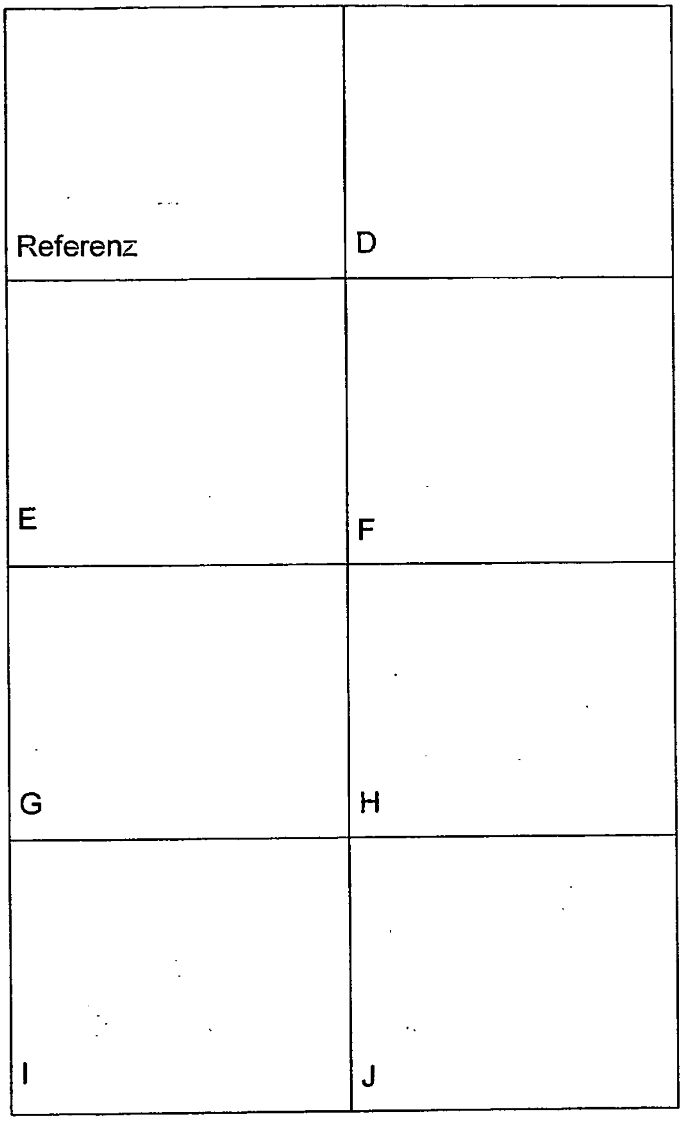

Abbildung 3

| | |
|---|---|
| Muster J | Muster J γ-steril |

Abbildung 4

| | |
|---|---|
| Muster G | Muster G:<br>Lagerung 6 Monate bei 50°C |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19958458 A1
- EP 1116698 A1
- EP 1116700 A1
- EP 1159972 A1
- WO 0009173 A
- US 5681575 A
- WO 02062403 A
- WO 02078755 A
- US 6143318 A
- GB 2178422 A
- DE 10213632
- US 5470585 A
- WO 9624364 A
- EP 1116700 A
- WO 9743328 A1
- DE OS3103499 A
- DE OS3103500 A
- EP 0147588 A1
- EP 0665856 B1
- DE 19618825 A1
- DE 19618825
- DE 10056010
- DE 10056011
- DE 10142918
- DE 10114382
- EP 076657 A1
- WO 9855148 A
- EP 0579435 A
- EP 0392608 A
- EP 0756493 A
- DE 3713601 A1
- DE 2743979 C3
- DE 10121092


### In der Beschreibung aufgeführte Nicht-Patentliteratur


- **M.C.Robson.** Clinical Research can improve the outcome of treatment of problem wounds : Infection as a paradigm. *8th Annual Meeting of the ETRS,* 27. August 1998
- **McMurry LM et al.** *FEMS Microbiol Lett,* 1998, vol. 166 (2), 305-9
- **Cookson BD et al.** *Lancet,* 1991, vol. 337 (8756), 1548-9
- **Uhl S.** *Lancet,* 1993, vol. 342 (8865), 248
- Polyurethanes, Chemistry and Technology. **Saunders ; Frisch.** High Polymers. Interscience Publishers, 1962, vol. 1, XVI, 32-42
- **Becker ; Braun.** Kunststoff-Handbuch, Bd. 7, Polyurethane. Carl-Hauser, 1983, vol. 7, 121 ff
- **H.P.Fiedler.** Lexilcon der Hilfsstoffe für Pharmazie, Kosmetik und angrenzende Gebiete. 1989, 293
- **K. Uekama et al.** Cyclodextrin drug carrier systems. *Chemical Reviews,* 1998, vol. 98, 2045-2076
- **T. Loftsson.** Cyclodextrins: new drugdelivery systems in dermatology. *Int. J. Dermatology,* vol. 37, 241-246
- **J. Zatz et al.** Applications of cyclodextrins in skin products. *Cosmetics & Toiletries,* vol. 112, 39ff
- **U. Citemesi.** *Cosmetics & Toiletries,* vol. 110, 53 ff